# EUROPEAN PATENT APPLICATION

(11) **EP 4 772 180 A1**
(43) Date of publication of application: **08.07.2026**
(21) Application number: 25150225.8
(22) Date of filing: 03.01.2025
(51) Int. Cl.: A61K 31/55, A61P 25/00, A61P 25/24, A61P 27/02, A61P 29/00

(54) **DIBENZAZEPINE DERIVATIVES, PHARMACEUTICAL COMPOSITIONS AND USES**

(71) Applicant: Shenzhen Evergreen Therapeutics Co., Ltd., Shenzhen, Guangdong 518107 (CN)
(72) Inventor: DU, Xin, Shenzhen, 518107 (CN); LI, Shanshan, Shenzhen, 518107 (CN); LIU, Xuhan, Shenzhen, 518107 (CN); HU, Tao, Newton, 02464 (US); DU, Tao Tom, Shenzhen, 518107 (CN)
(74) Representative: J A Kemp LLP

(57) **Abstract**

This invention relates to the field of medical technology, specifically involving a compound and its pharmaceutical composition and use for improving the bioavailability of desipramine. Pharmaceutical compositions containing the compound and said compound are capable of increasing the bioavailability of desipramine.

## Description

### Technical Fields

This invention relates to the field of pharmaceutical technology, specifically involving a compound and its pharmaceutical composition and use for improving the bioavailability of desipramine.

### Background

Desipramine is an antidepressant that blocks the recovery of monoamine neurotransmitters such as norepinephrine or serotonin. Used to treat all kinds of depression. The existing common dosage forms of desipramine hydrochloride are tablets, the specifications are: 25mg, 50mg. Oral absorption is easy from the gastrointestinal tract, absorption is not affected by food. It is widely distributed in the body, easily penetrates the blood-cerebrospinal fluid barrier, and accumulates in the brain. There is a significant positive correlation between blood concentration and clinical antidepressant efficacy, and healthy people can obtain better effects only when blood concentration is above 115-155 µg/L. The peak time of desipramine is 4 ~ 6h, which is mainly metabolized in the liver.

Start with 25mg orally 3 times a day; Increase to 50mg 3 to 4 times a day; Patients with severe depression can reach 300mg per day. There are problems such as low bioavailability, large dosage and low patient compliance, which affect the therapeutic effect of the disease.

### Summary of the Invention

According to one aspect of the invention, the invention provides a compound for improving the bioavailability of desipramine and a pharmaceutical composition and use thereof, which can improve the bioavailability of desipramine. 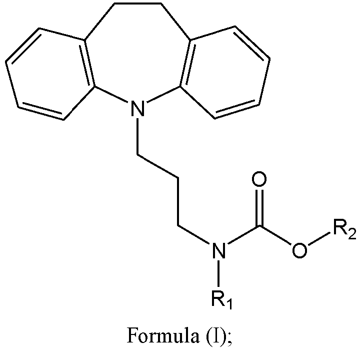 Where R₁ is chosen from H, alkyl of C1-C4, alkoxy of C1-C4, or cycloalkyl of C3-C6;
R₂ is selected from an unsubstituted or substituted alkyl of C1-C4, an aryl of C5-C20, or 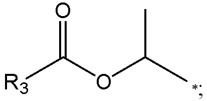
R₃ is selected from the alkyl of unsubstituted or substituted C1-C4 or the aryl of C5-C20;

In the group definition of R₂ and R₃, "Substituted" means that 1-3 H of the group is replaced by at least one of the selected from hydroxyl, amino, carboxyl, unsubstituted or substituted alkyl groups of C1-C4, unsubstituted or substituted aryl of C5-C20, unsubstituted or substituted 3~20 membered heterocyclic groups, and gelled groups and 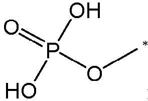 formed by unsubstituted or substituted 3~20 membered heterocyclic groups and aromatic rings of C5-C20. Among them, the substituent group of the substituted alkyl group of C1-C4, the aryl group of C5-C20, the heterocyclic group of 3~20 member, the cohesive group formed by heterocyclic group of 3~20 member and the aromatic ring of C5-C20 is alkyl or hydroxyl group of C1-C4. The heteroatom of the heterocyclic group is N, O or S, and the number of heteroatoms is 1-6.

Unless otherwise specified, the following definitions are used to illustrate and define the meaning and scope of the various terms used herein to describe the present invention.

The following definitions of general terms apply whether they occur alone or in combination.

Any open valence bond occurring on the carbon, oxygen, sulfur, or nitrogen atoms in the structure given in this article indicates the presence of hydrogen atoms.

Unless otherwise specified, the term "substituted" means that the specified group may have 1, 2, 3, 4, 5, or 6 substituents. When more than one substituent can be had on the group and multiple possible substituents are given, the substituents are selected independently and need not be identical.

The term "unsubstituted" refers to the absence of a substituent on a specified group.

The term "substituted" means that the specified group is replaced by one or more substituents independently selected from possible substituents.

When specifying the number of substituents, the term "one or more" refers to the maximum number of possible substituents from one to the other, i.e., substituting one hydrogen to the point where all the hydrogens are replaced by the substituent. 1, 2, 3, 4, or 5 substituents are preferred unless otherwise specified.

The term "oxy" refers to the -O- group. The term "carboxyl" refers to the -C(=O)OH group. "Amino" means the -NH₂ group.

The term "alkyl" refers to straight or branched chains, such as saturated hydrocarbon groups with 1-4 carbon atoms. The term "unsubstituted or substituted C1-C4 alkyl" is divided into unsubstituted alkyl and substituted alkyl groups. Unsubstituted alkyl refers to alkyl groups that have not been replaced by substituents. Examples of "unsubstituted alkyl groups" include methyl, ethyl, n-propyl, isopropyl, n-butyl, isobutyl, etc. Substituted alkyl refers to an alkyl group replaced by 1-3 substituents, such as: the hydroxyl, amino, carboxyl, unsubstituted or substituted alkyl groups of C1-C4, unsubstituted or substituted aryl groups of C5-C20, unsubstituted or substituted heterocyclic groups of 3 to 20 memberes, unsubstituted or substituted heterocyclic groups of 3 to 20 memberes and the aromatic ring of C5-C20 form a cohesive base and 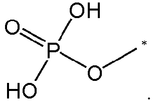 Among them, the substituted alkyl group of C1-C4, the aryl group of C5-C20, the heterocyclic group of 3-20, the substituted alkyl group of 3~20 heterocyclic group and the condensed group of the aromatic ring of C5-C20 are the alkyl group or hydroxyl group of C1-C4.

The term "cycloalkyl" refers to a non-aromatic, saturated or unsaturated cyclic hydrocarbon group composed of carbon and hydrogen atoms, which may include a condensed ring system, a bridge ring system or a spiro ring system. Such as, but not limited to, cyclopropyl, cyclobutyl, and cyclopentyl.

The term "aryl" or "aromatic ring" refers to a conjugated hydrocarbon ring system group having, for example, 5 to 20, preferably 5 to 10 carbon atoms in the ring portion. Aryl groups usually include but are not limited to the following groups: benzene, naphthalene, anthracene, biphenyl, 1, 2-dihydronaphthalene, 1,2,3, 4-tetrahydronaphthalene, etc.

The term "heterocyclic group" or "heterocyclic group" refers to a stable 3 - to 20-membered aromatic or non-aromatic ring group consisting of 1 to 6 heteroatoms selected from nitrogen, oxygen, and sulfur, which may be saturated or unsaturated. Such as, but not limited to, pyrrolidyl, tetrahydrofuranyl, 2(5H) furanone, tetrahydropyranyl, morpholinyl, thiomolinyl, piperazinyl, homopiperazinyl, propylene oxide, imidazolidyl, 1, 3-dioxacyclopentene-2-one, 1, 3-dioxacyclopentene-2-one, 1, 3-dioxacyclohexane-2-one, n-pyridinyl urea, pyrimidinyl And 1, 1-dioxy-thiopolinyl.

The term "3 to 20 membered heterocyclic group formed with the aromatic ring of C5-C20" refers to the aggregation of aromatic and heterocyclic rings, for example 1-3 aromatic rings with 1-3 single heterocyclic rings. For example, but not limited to benzopyridine, benzopyrrole, or pyridino-isoquinoline.
* Indicates the linking site.

Compounds of the present invention may contain asymmetric centers or chiral centers, and therefore different stereoisomer forms exist. All stereoisomer forms of the compounds of the invention, including, but not limited to, diastereomers, enantiomers, and steric isomers, and mixtures of them such as racemic mixtures, will form part of the invention. In this article, all stereoisomers are considered when the stereochemistry of any particular chiral atom is not determined. In addition, the invention relates to all geometric and positional isomers. The compounds of the invention may exist in different tautomer forms, and all of these forms are included within the scope of the invention. All stereoisomers of the compounds of the present invention are expected to include mixture forms or pure or substantially pure forms. They may be resolved by physical methods such as fractional crystallization, separation or crystallization of diastereomeric derivatives, or by chiral column chromatographic separation.

The term "precursor drug" is a functional derivative of a compound of formula (I) that is readily converted into a compound of formula (I) in the body. A suitable derivative of formula may be selected and prepared by conventional techniques well known to those skilled in the art.

The term "pharmaceutically acceptable salt" as used herein refers to pharmaceutically acceptable organic or inorganic salts of the compounds of the present invention. Examples of salts include, but are not limited to, sulfate, citrate, acetate, oxalate, chloride, bromide, iodide, nitrate, acid sulfate, isonicotinate, lactate, salicylate, acid citrate, succinate, maleate, fumarate, gluconate, formate, mesylate and bamoate. "Pharmaceutically acceptable salt" may refer to the inclusion of another molecule such as maleate or other equilibrium ion. The equilibrium ion stabilizes the charge in the parent compound. A "pharmaceutically acceptable salt" may have more than one charged atom, and multiple charged atoms may have multiple equilibrium ions.

If the compound of the invention is a base, the required "pharmaceutically acceptable salt" may be prepared by a suitable method, for example, by treating the free base with the following inorganic acids: hydrochloric acid, hydrobromic acid, sulfuric acid, nitric acid, phosphoric acid; Or organic acids such as acetic acid, maleic acid, succinic acid, mandelic acid, fumaric acid, malonic acid, pyruvate, salicylic acid, glucuronic acid or galacturonic acid, alpha-hydroxy acid such as citric acid or tartaric acid, amino acid such as glutamic acid, aromatic acid such as benzoic acid or cinnamic acid, sulfonic acid such as mesylate or p-toluenesulfonic acid.

If the compound of the present invention is an acid, the required "pharmaceutically acceptable salt" may be prepared by a suitable method, for example, by treating the free acid with an inorganic or organic base such as an amine, an alkali metal hydroxide or an alkaline earth metal hydroxide. Examples of suitable salts include, but are not limited to, organic salts derived from amino acids; salts of primary, secondary, and tertiary amines; and salts of cyclic amines such as piperidine, morpholine, and piperazine; and inorganic salts derived from sodium, calcium, potassium, magnesium, manganese, iron, copper, zinc, aluminum, and lithium.

The compound of the present invention can exist in a continuous solid state ranging from completely amorphous to completely crystalline. The term "amorphous" refers to a state in which the material lacks long-range orderliness at the molecular level and, depending on temperature, can exhibit physical properties of a solid or liquid. Typically, such materials do not give a distinct X-ray diffraction pattern, and are more formally described as liquid while exhibiting solid properties. When heated, a change from solid to liquid properties occurs and is characterized by a change in state, usually of second order (" glass transition "). The term "crystal" refers to a solid phase in which the material has a regular ordered internal structure at the molecular level and gives a unique X-ray diffraction pattern with defined peaks. Such a material will also exhibit the properties of a liquid when fully heated, but the change from solid to liquid is characterized by a phase transition, usually of first order (" melting point ").

The term "polycrystalline substance" as used herein refers to the different solid crystalline phases resulting from the presence of two or more different molecular arrangements in the solid state of certain compounds of the present invention. Certain compounds of the present invention may exist in more than one crystalline form, and the present invention is intended to include various crystalline forms and mixtures thereof.

The term "solvate" as used herein refers to a combination or complex of one or more solvent molecules with the compound of the present invention. Examples of solvents that form solvocomplexes include, but are not limited to, water, isopropyl alcohol, ethanol, methanol, dimethylsulfoxide, ethyl acetate, acetic acid, and ethanolamine. The compounds of the invention may exist in non-solvated form or in solvated form with pharmaceutically acceptable solvents such as water, ethanol, etc., so the invention will include both solvated and non-solvated forms.

The compound of the invention may contain an unnatural proportion of atomic isotopes on one or more of the atoms constituting the compound, and the term " isotopologue" has the same atomic number, but its atomic mass or mass number is different from the atomic mass or mass number that predominates in nature. For example, radioisotopes may be used to label compounds such as deuterium (2H), tritium (3H), iodine-125 (125I) or C-14 (14C). All transformations of the isotopic composition of the compounds of the present invention, whether radioactive or not, are included within the scope of the present invention. Isotopic variants may enhance certain therapeutic advantages, such as deuterium enrichment that can increase in vivo half-life or reduce dose requirements, or that can provide standard compounds that can be used for characterization of biological samples. Isotopically enriched compounds in formula (1) may be prepared by conventional techniques well known to those skilled in the art, or by methods similar to those described in the routes and embodiments herein, using appropriate isotopically enriched reagents and/or intermediates, without excessive experimentation.

In some preferred embodiments, R₂ is chosen from the substituted or unsubstituted alkyl, phenyl of C1-C4 or 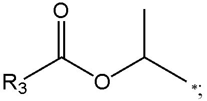 where the term R₃ is defined as above.

In some preferred embodiments, R₂ is selected from an unsubstituted or substituted alkyl group of C1-C4, substituted when the 1-3 H on the alkyl group is replaced by at least one of the hydroxyl groups, 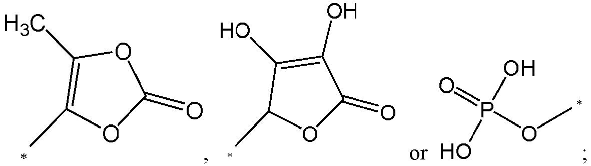 and preferably, R₂ is selected from the substituted methylene group replaced by

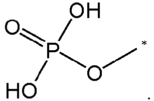

In some preferred embodiments, the compound has the structural formula shown in formula II: 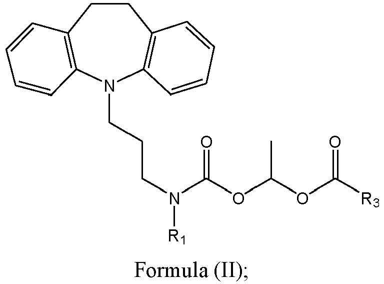 Where R₁ and R₃ are defined above.

In some preferred embodiments, R₃ is selected from an unsubstituted or substituted alkyl or phenyl group of C₁-C₄, substituted when the 1-3 H on the alkyl group is replaced by at least one of the selected amino groups, carboxyl groups or 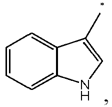 and preferably by an amino group or a carboxyl group.

In some preferred embodiments, R₁ is chosen from H, an alkyl group of C1-C4, preferably methyl. The compound is chosen from any of the following compounds:

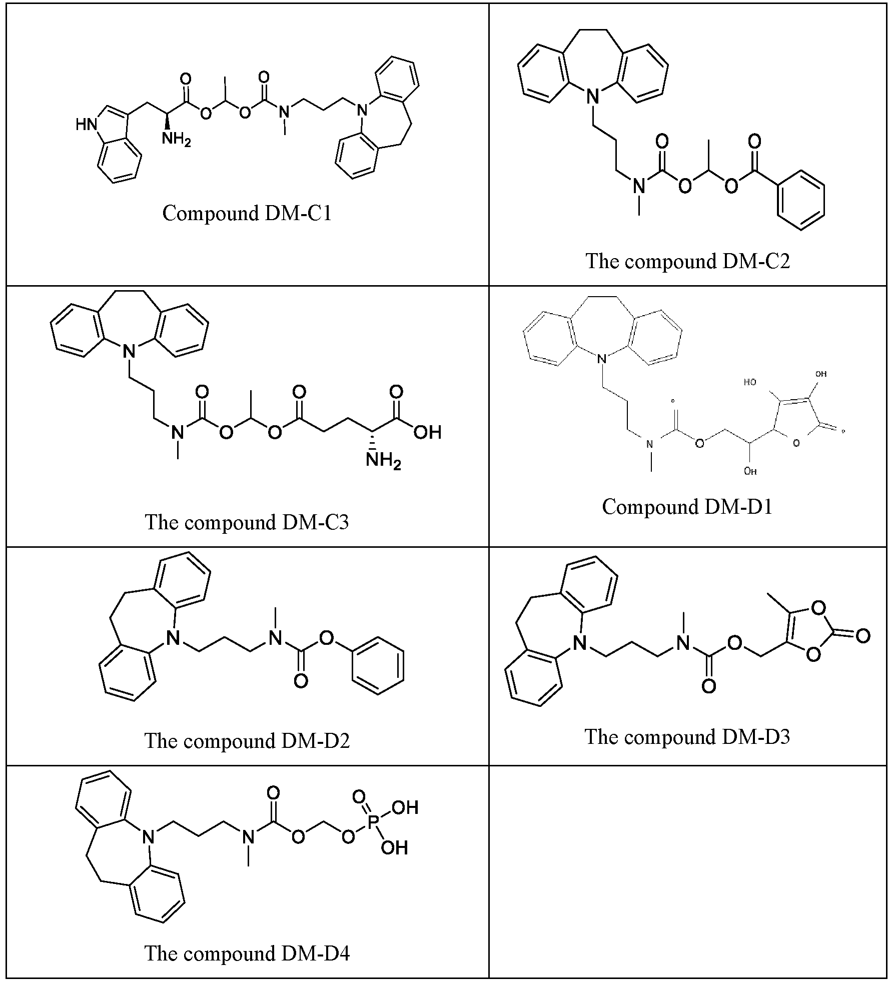

The invention also provides a pharmaceutical composition comprising a compound described in any item of the invention or at least one of its pharmaceutically acceptable salts, stereoisomers, tautomers, precursor drugs, amorphous substances, isotopic bodies, polycrystalline forms or solvocomplexes, and pharmaceutically acceptable carriers and/or adjuvants.

The compounds used in the present invention may therefore be combined with conventional additives or diluents to form the pharmaceutical composition and its unit dose form. Such forms include solids (especially tablets, filled capsules, powders, and pills), liquids (especially aqueous or non-aqueous solutions, suspensions, emulsion), or capsules filled with the above forms, all forms of oral administration, suppositories for rectal administration, poultices for transdermal administration, eye drops for ocular administration, and sterile injectable solutions for parenteral administration. Such pharmaceutical compositions and their unit dose forms may include regular ingredients in regular proportions, with or without additional active compounds or ingredients, and such unit dose forms may contain any suitable effective amount of active ingredient commensurate with the required daily application dose range.

The compounds used in the present invention may be administered in a variety of oral and parenteral dosage forms. The following dosage forms may contain compounds of the invention as active ingredients or pharmaceutically acceptable salts thereof for persons skilled in the field of the present invention.

Pharmaceutically acceptable carriers may be solids or liquids to make pharmaceutical compositions of compounds used in the present invention. Preparations in solid form include powders, tablets, nine doses, capsules, flat capsules, suppositories, and dispersible granules. Solid carriers may be one or more substances that also act as diluents, flavorings, solubilizers, lubricants, suspensions, adhesives, preservatives, tablet disintegrators, or encapsulated materials.

In a powder, the carrier is a subdivided solid, which is mixed with subdivided active ingredients.

In tablets, the active ingredient is mixed with a carrier with the necessary bonding properties in proper proportions and compressed into the desired shape and size.

Powders and tablets preferably contain 5% or 10% to about 70% of the active compound. Suitable carriers are magnesium carbonate, magnesium stearate, talc powder, sugar, lactose, pectin, dextrin, starch, gelatin, tragacanth, methyl cellulose, sodium carboxymethyl cellulose, wax with low melting point, cocoa butter, etc. The term "preparation" includes an active compound containing an active compound formulated with a encapsulated material acting as a carrier, which provides a sac in which the active ingredient with or without a carrier is surrounded by a carrier and thus bonded to it. Tablets, powder, capsules, pills may be used as solid forms suitable for oral administration.

To prepare suppositories, a wax with a low melting point, such as a purecompound of fatty acid glycerides or cocoa butter, is first melted, then the active ingredient is evenly dispersed in it by stirring. This uniform molten mixture is then poured into a suitably sized mold, allowed to cool and thus solidify.

Compositions suitable for vaginal administration may be available in the form of vaginal suppositories, cotton plugs, creams, gels, pastes, foams, or sprays containing suitable carriers known in the art in addition to the active ingredients.

Liquid preparations include solutions, suspensions, and emulsions such as, for example, aqueous solutions or water-propylene glycol solutions. For example, parenteral injectable liquid preparations can be formulated as a solution of water-polyethylene glycol.

Thus, the compound used in the present invention may be formulated into a preparation for parenteral administration (e.g. injection, such as rapid concentration or continuous infusion), and may be present together with added preservatives in unit doses in ampoules, pre-filled syringes, small volume infusion bags, or multi-dose containers. The composition may take the form of a suspension, solution or emulsion of an oil-based or water-based carrier, and may contain preparation components such as a suspension, stabilizer and/or dispersant. Alternatively, the active ingredient may be in the form of a powder, which may be obtained by sterile solid aseptic separation or by freeze-drying of a solution, for pre-clinical reconstruction with a suitable carrier such as sterile, pyrogen free water.

Aqueous solutions suitable for oral administration may be prepared by dissolving the active ingredient in water and adding the desired colorants, flavorings, stabilizers, and thickeners.

Aqueous suspensions suitable for oral administration may be prepared by dispersing subdivided active ingredients in water containing viscous substances such as natural or synthetic gums, resins, methyl cellulose, sodium carboxymethyl cellulose, or other known suspensions.

It also includes solid preparations designed to be converted into liquid preparations for oral administration shortly before their immediate use. Such liquid preparations include solutions, suspensions, and emulsions. In addition to the active ingredients, such preparations may contain colorants, flavorings, stabilizers, buffers, artificial and natural sweeteners, dispersants, thickeners, solvants, etc.

In order to apply topically to the epidermis, the compounds of the invention may be formulated as a ointment, cream or lotion or a transdermal patch. For example, plasters and creams may be formulated from water-based or oil-based substrates with the addition of suitable thickeners and/or gelling agents. Lotions may be formulated from an aqueous or oil-based base and usually contain one or more emulsifiers, stabilizers, dispersants, suspensions, thickeners or colorants.

The solution or suspension may be applied directly to the nasal cavity by conventional means such as an eyedropper, straw, or sprayer. The composition may be in the form of a single or multiple dose.

Respiratory administration may also be achieved by aerosol, in which the active ingredient is packed in pressurized packages with a suitable propellant including chlorofluorocarbons (CFCS) such as dichlorodifluoromethane, trichlorofluoromethane or dichlorotetrafluoroethane, carbon dioxide or other suitable gases. Aerosols may also contain surfactants, such as lecithin, as appropriate. The dosage of the drug can be controlled via a dosing valve.

In compositions intended for respiratory tract administration (including compositions for intranasal use), typically the compound has a small particle size, such as a size of 5 microns or less. Such particle size may be obtained by methods known in the art, such as by micropulverization.

Compositions suitable for the slow release of the active ingredient may be applied if needed.

Tablets or capsules intended for oral administration and liquids intended for intravenous administration, as well as continuous infusion, are preferred compositions.

More detailed information on formulations and delivery technologies can be found in the latest edition of Remington's Pharmaceutical Sciences(Maack Publishing Co.,Easton, PA).

The amount of active component in a unit dose formulation can vary depending on the specific application and potency of the active component, and can be adjusted from 0.01mg to approximately 0.1g. For example, in pharmaceutical use, the drug may be administered three times a day in capsules of 0.01 to approximately 100mg, and the composition may also contain other compatible therapeutic agents if necessary.

### Therapeutic Approaches

In therapeutic uses, compounds used in the present invention are used in starting doses of 0.001mg/kg to 10mg/kg body weight per day. However, these doses may vary according to the needs of the patient, the severity of the condition being treated, and the compound used. In general, treatment is started at a smaller dose than the optimal dose of the compound, thereafter this dose is increased in small amounts for the best effect, and for convenience the total daily dose may be subdivided into multiple doses over the day if needed.

The use of any of the compounds or their stereoisomers, tautomers, precursor drugs, pharmaceutically acceptable salts, amorphous substances, polycrystalline substances, solvers, or pharmaceutical compositions in the preparation of drugs for the prevention, treatment, or relief of at least one of the following conditions: depression, pain, dry age-related macular degeneration disease, and neuropathy.

The use of any of the compounds or their stereoisomers, tautomers, precursor drugs, pharmaceutically acceptable salts, amorphous substances, polycrystalline substances, solvocomplexes, or pharmaceutical compositions in the preparation of drugs for improving the bioavailability of desipramine or its pharmaceutically acceptable salts.

Beneficial effects:
The compound provided by the invention to improve the bioavailability of desipramine can be metabolized to desipramine in the body, and can improve the bioavailability of desipramine and reduce the dosage compared with desipramine hydrochloride. And improve metabolic stability.

### Brief Description of the Drawings

In order to make the content of the invention easier to be clearly understood, the following is a further detailed description of the invention according to specific embodiments of the invention combined with the attached drawings, in which,

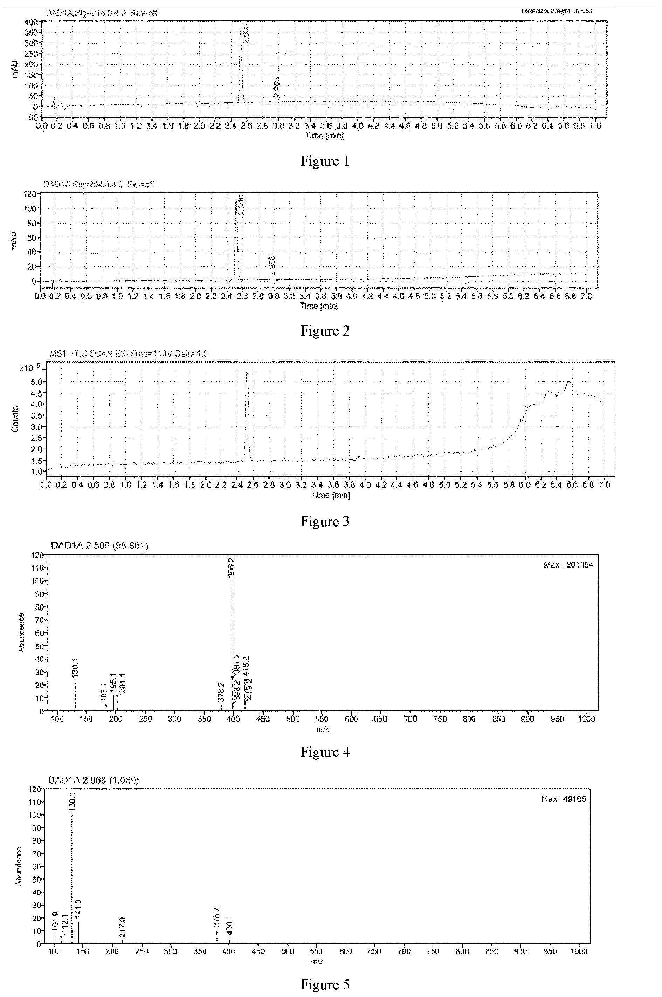

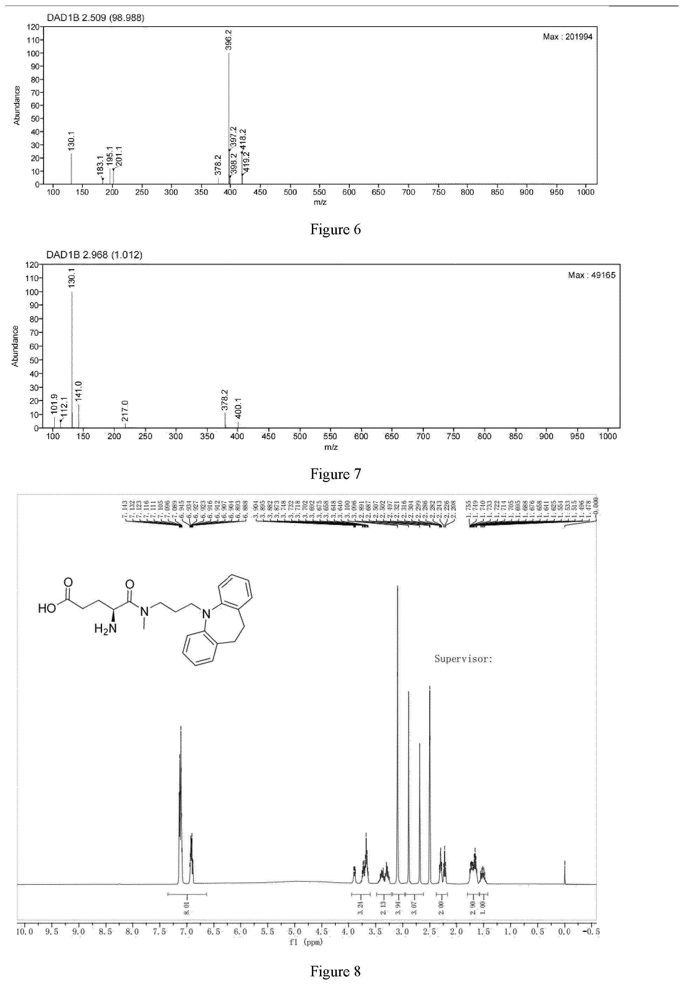

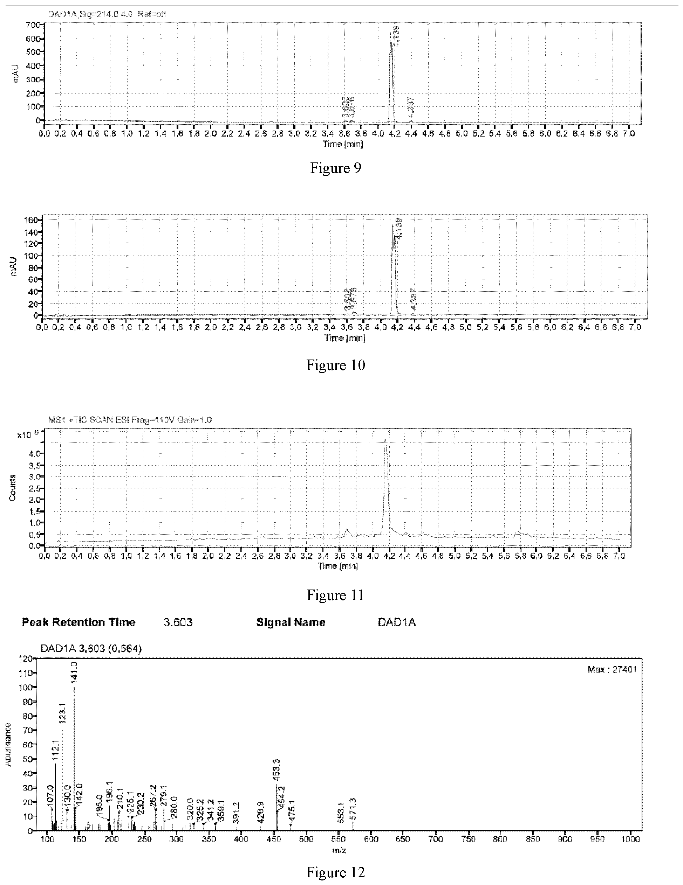

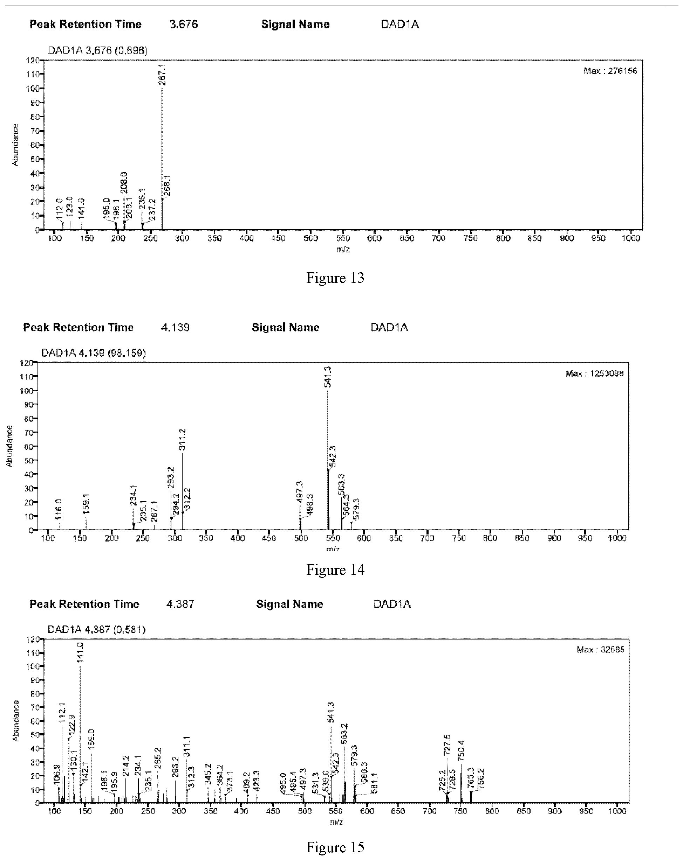

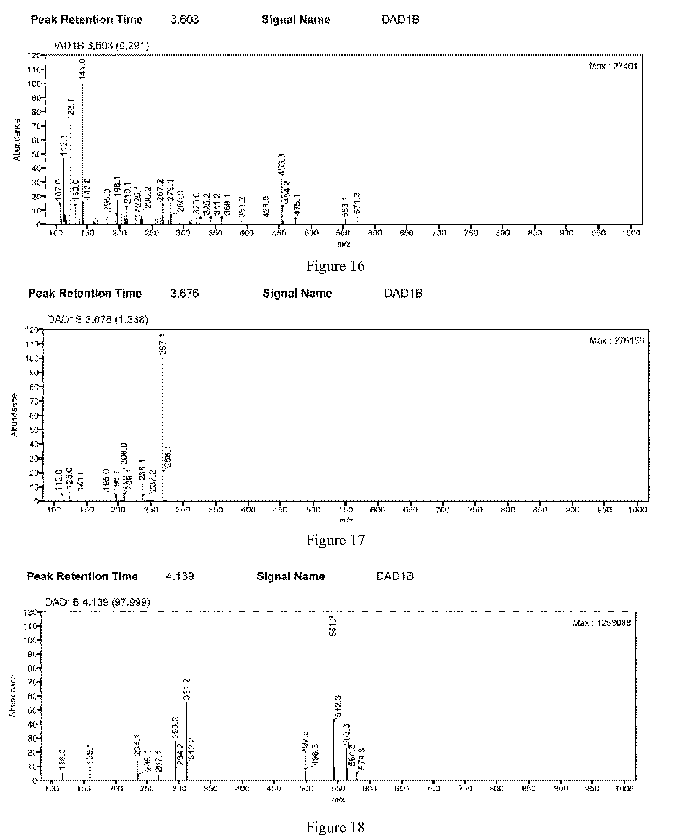

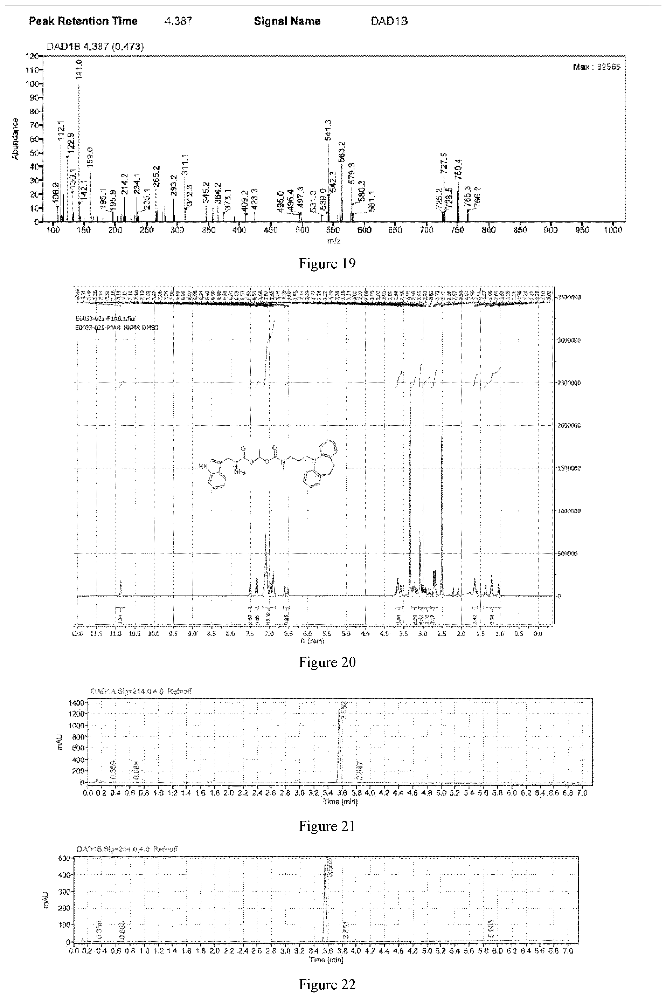

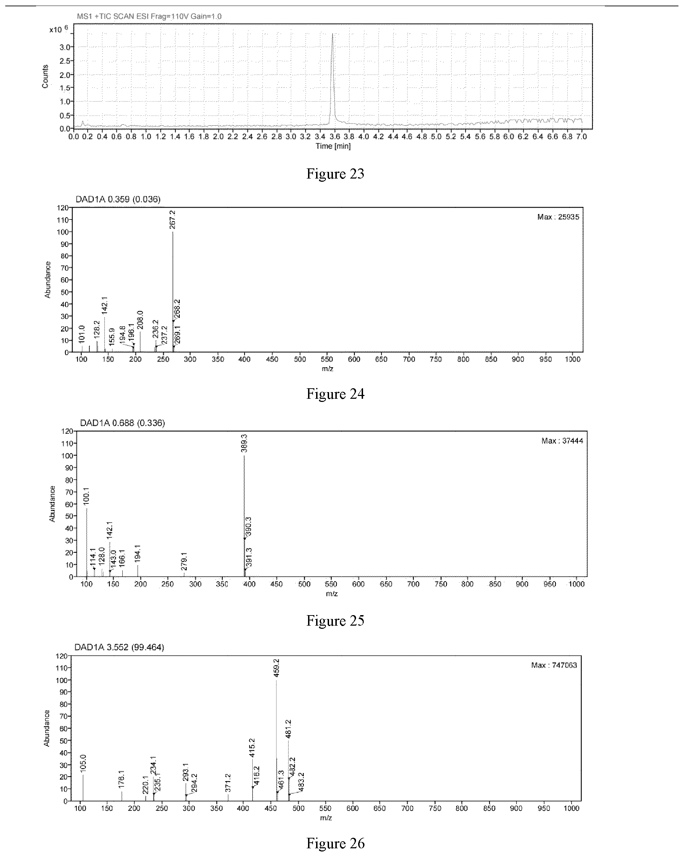

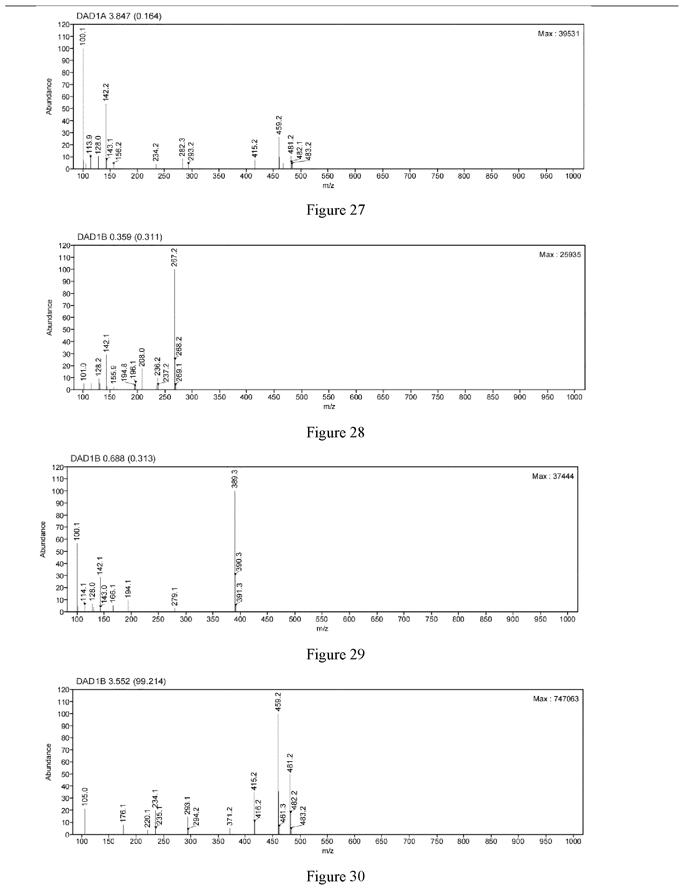

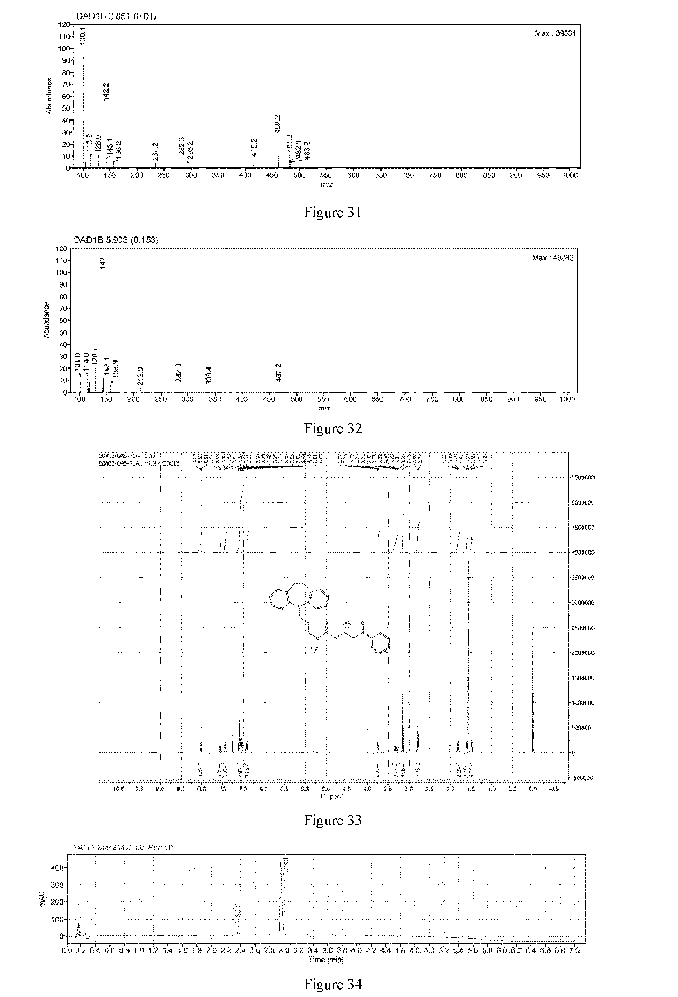

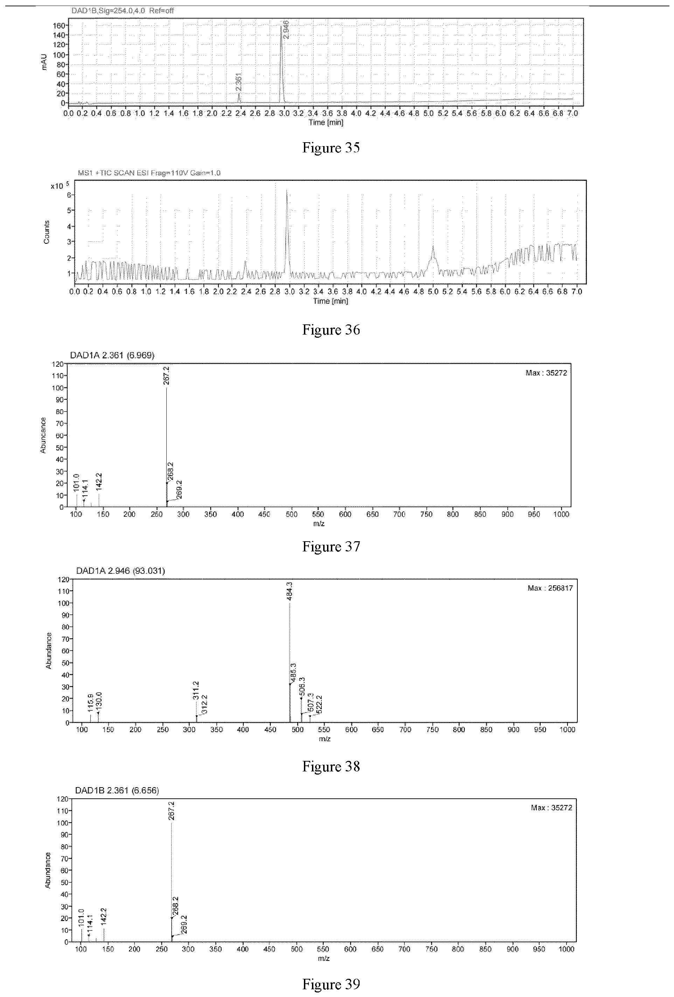

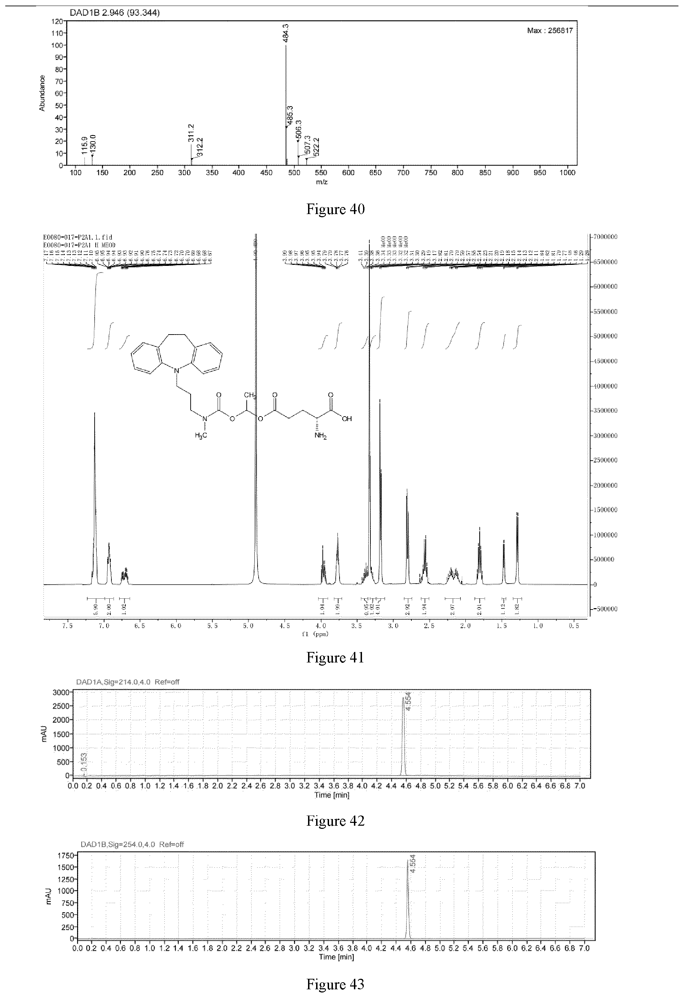

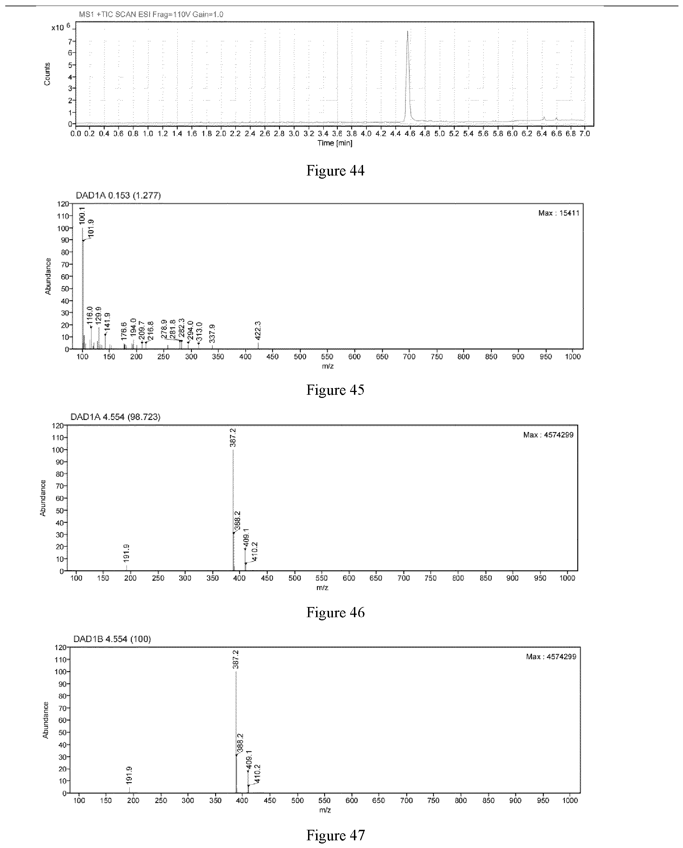

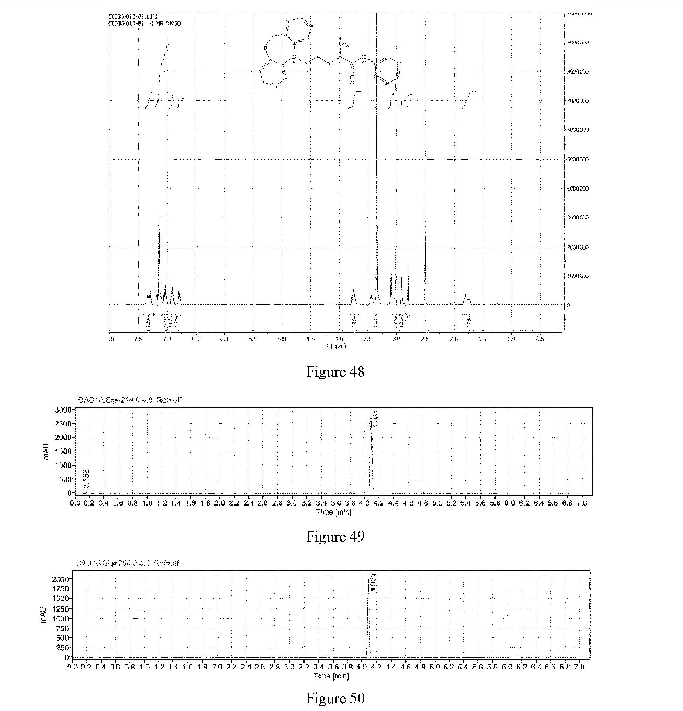

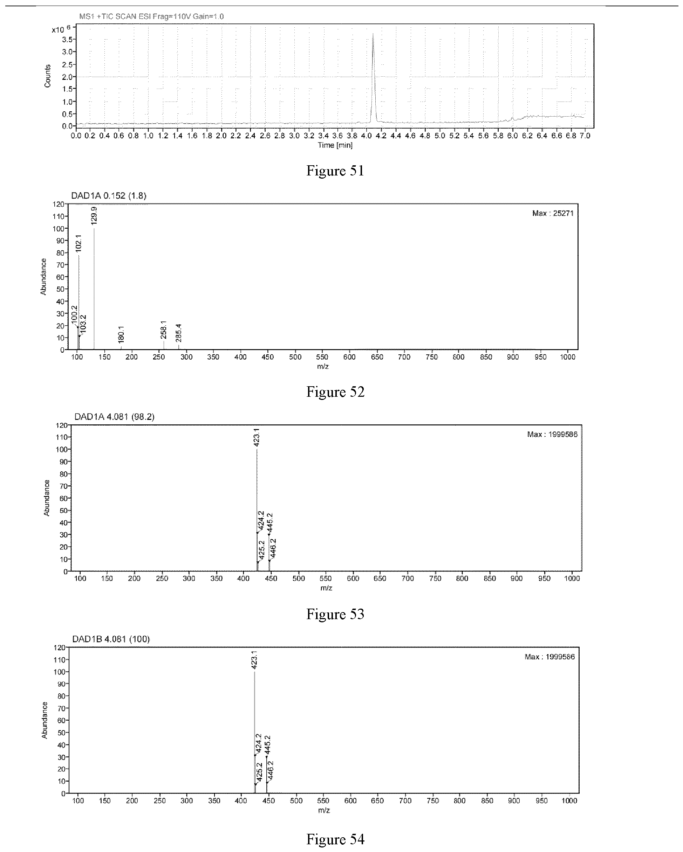

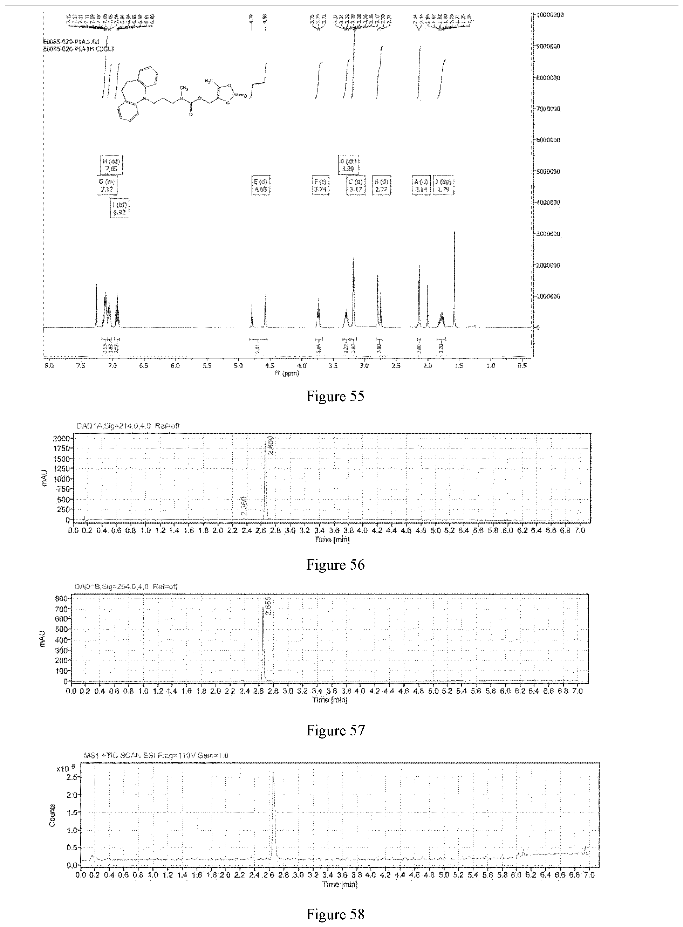

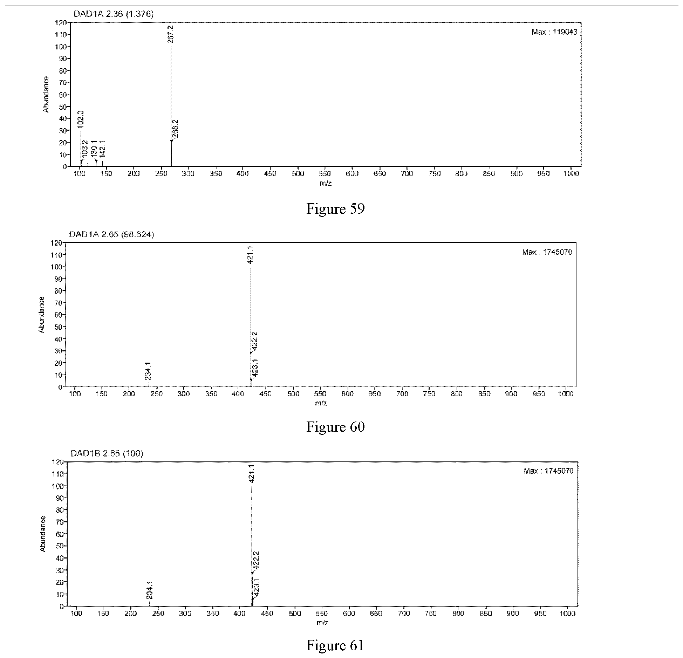

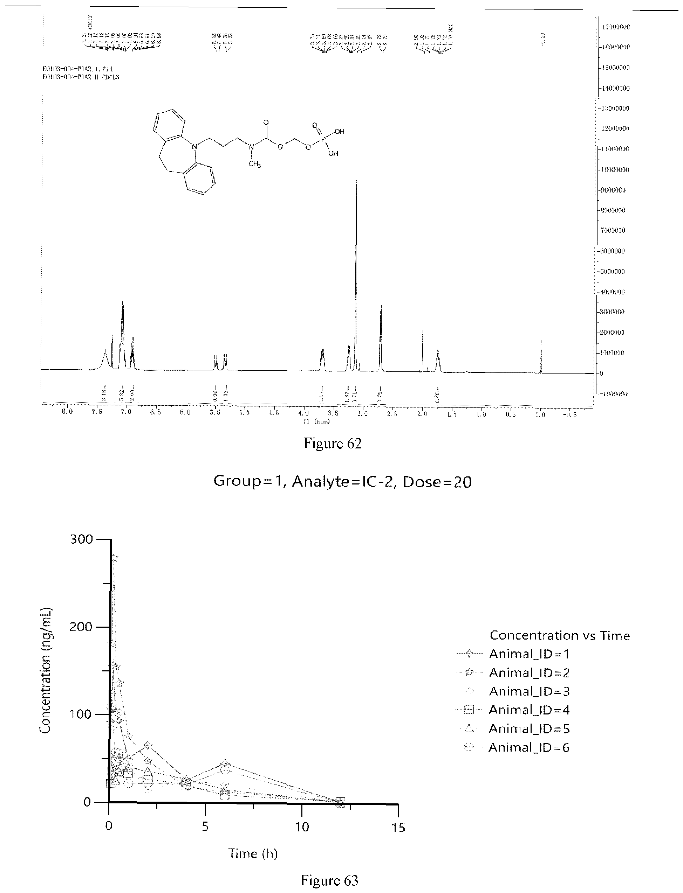

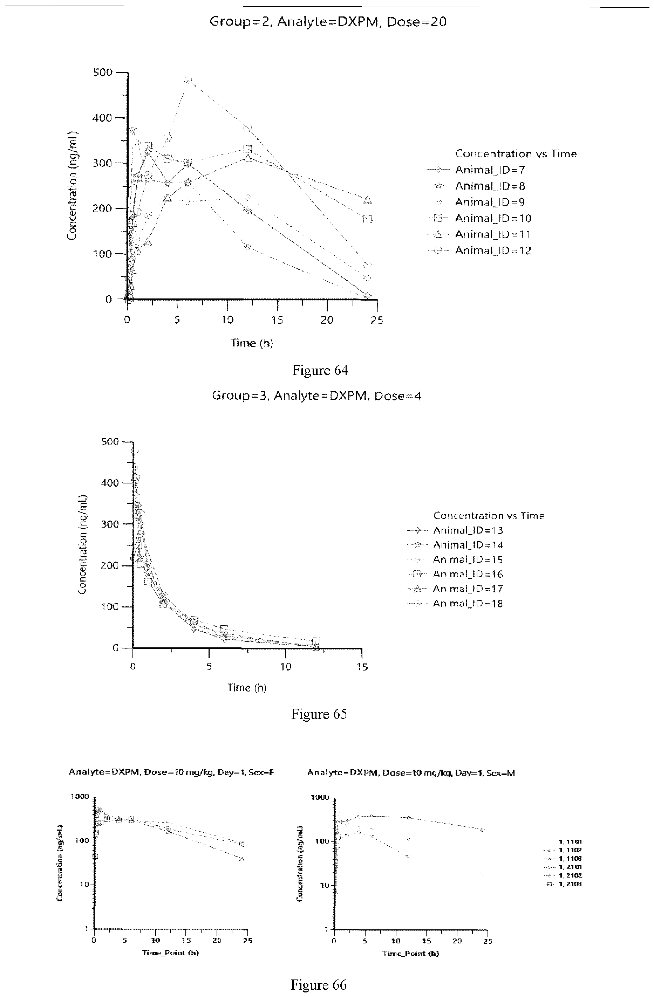

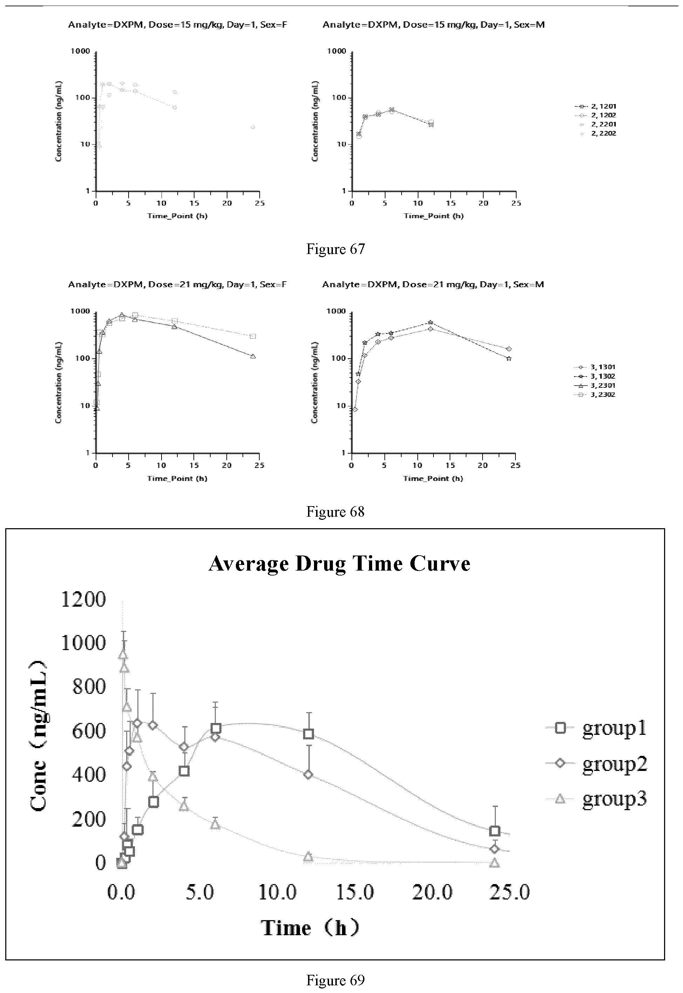

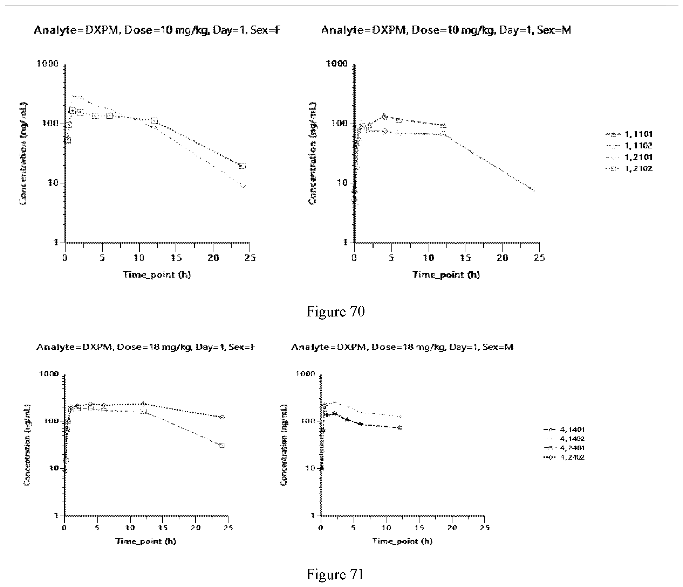

Figure 1 shows the UV absorption chromatogram of compound DM-A1 at 214nm. FIG. 2 shows the UV absorption chromatogram of compound DM-A1 at 254nm. FIG. 3 shows the mass spectrum of compound DM-A1. Figure 4 shows the mass spectrum of compound DM-A1 214nm RT 2.509; Figure 5 shows the mass spectrum of compound DM-A1 214nm RT 2.968; Figure 6 shows the mass spectrum of compound DM-A1 254nm RT 2.509; Figure 7 shows the mass spectrum of compound DM-A1 254nm RT 2.968; FIG. 8 shows the NMR spectra of compound DM-A1. FIG. 9 shows the UV absorption chromatogram of compound DM-C1 at 214nm. FIG. 10 shows the UV absorption chromatogram of compound DM-C1 at 254nm. FIG. 11 shows the mass spectrum of compound DM-C1. FIG. 12 shows the mass spectrum of compound DM-C1 214nm RT 3.603. Figure 13 shows the mass spectrum of compound DM-C1 214nm RT 3.676; FIG. 14 shows the mass spectrum of compound DM-C1 214nm RT 4.139; FIG. 15 shows the mass spectrum of compound DM-C1 214nm RT 4.387; FIG. 16 shows the mass spectrum of compound DM-C1 254nm RT 3.603; FIG. 17 shows the mass spectrum of compound DM-C1 254nm RT 3.676; Figure 18 shows the mass spectrum of compound DM-C1 254nm RT 4.139; FIG. 19 shows the mass spectrum of compound DM-C1 254nm RT 4.387; FIG. 20 shows the NMR spectra of compound DM-C1. FIG. 21 shows the UV absorption chromatogram of compound DM-C2 at 214nm. FIG. 22 shows the UV absorption chromatogram of compound DM-C2 at 254nm. FIG. 23 shows the mass spectrum of compound DM-C2. FIG. 24 shows the mass spectrum of compound DM-C2 214nm RT 0.359; FIG. 25 shows the mass spectrum of compound DM-C2 214nm RT 0.688; FIG. 26 shows the mass spectrum of compound DM-C2 214nm RT 3.552; FIG. 27 shows the mass spectrum of compound DM-C2 214nm RT 3.847; FIG. 28 shows the mass spectrum of compound DM-C2 254nm RT 0.359; FIG. 29 shows the mass spectrum of compound DM-C2 254nm RT 0.688; FIG. 30 shows the mass spectrum of compound DM-C2 254nm RT 3.552; Figure 31 shows the mass spectrum of compound DM-C2 254nm RT 3.851; FIG. 32 shows the mass spectrum of compound DM-C2 254nm RT 5.903; FIG. 33 shows the NMR spectra of compound DM-C2. Figure 34 shows the UV absorption chromatogram of compound DM-C3 at 214nm. Figure 35 shows the UV absorption chromatogram of compound DM-C3 at 254nm. FIG. 36 shows the mass spectrum of compound DM-C3. Figure 37 shows the mass spectrum of compound DM-C3 214nm RT 2.361. Figure 38 shows the mass spectrum of compound DM-C3 214nm RT 2.946; FIG. 39 shows the mass spectrum of compound DM-C3 254nm RT 2.361. FIG. 40 shows the mass spectrum of compound DM-C3 254nm RT 2.964; FIG. 41 shows the NMR spectra of compound DM-C3. FIG. 42 shows the UV absorption chromatogram of compound DM-D2 at 214nm. Figure 43 shows the UV absorption chromatogram of compound DM-D2 at 254nm. FIG. 44 shows the mass spectrum of compound DM-D2. FIG. 45 shows the mass spectrum of compound DM-D2 214nm RT 0.153. FIG. 46 shows the mass spectrum of compound DM-D2 214nm RT 4.554; FIG. 47 shows the mass spectrum of compound DM-D2 254nm RT 4.554; FIG. 48 shows the NMR spectra of compound DM-D2. FIG. 49 shows the UV absorption chromatogram of compound DM-D3 at 214nm. FIG. 50 shows the UV absorption chromatogram of compound DM-D3 at 254nm. FIG. 51 shows the mass spectrum of compound DM-D3. FIG. 52 shows the mass spectrum of compound DM-D3 214nm RT 0.152; Figure 53 shows the mass spectrum of compound DM-D2 214nm RT 4.081; FIG. 54 shows the mass spectrum of compound DM-D2 254nm RT 4.081; FIG. 55 shows the NMR spectra of compound DM-D2. Figure 56 shows the UV absorption chromatogram of compound DM-D3 at 214nm. FIG. 57 shows the UV absorption chromatogram of compound DM-D3 at 254nm. FIG. 58 shows the mass spectrum of compound DM-D3. Figure 59 shows the mass spectrum of compound DM-D3 214nm RT 2.36; FIG. 60 shows the mass spectrum of compound DM-D3 214nm RT 2.65; FIG. 61 shows the mass spectrum of compound DM-D3 254nm RT 2.65; FIG. 62 shows the NMR spectra of compound DM-D3. FIG. 63 is the drug time curve (blood concentration time curve) of group 1 of experimental case 1. Figure 64 is the drug time curve of group 1, group 2, Figure 65 is the drug time curve of group 1, group 3, Figure 66 is the drug time curve of group 2, group 1, Figure 67 is the drug time curve of group 2, group 2, Figure 68 is the drug time curve of group 2, group 3, Figure 69 is the average drug time curve of group 1 to 3, FIG. 70 is the drug time curve of experiment case 4 group 1, and FIG. 71 is the drug time curve of experiment case 4 group 2.

### Detailed Description

The technical scheme of the invention is described clearly and completely below. Obviously, the embodiments described are partial embodiments of the invention, but not all embodiments. Based on the embodiments of the invention, all other embodiments obtained by ordinary technicians in the field without making creative labor fall within the scope of protection of the invention. Among them, the proportion of solvent used in the purification steps (e.g., preparative TLC, preparative chromatography, etc.) in the following embodiments is the volume ratio.

### Example 1

This embodiment is used to prepare the compound DM-C1, whose name is 1- (((3- (10, 11-dihydro-5h-dibenzo [b, f] aza-5-yl) propyl) (methyl) carbamoyl) oxy) ethyl L-tryptophan.

The synthesis route of the compound DM-C1 described in the present embodiment is shown below, specifically consisting of the following steps:

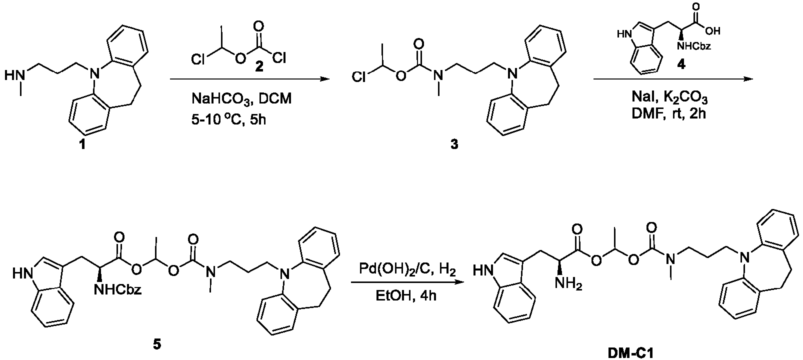

Step 1: Compound 1 (4.5g, 16.89mmol), sodium bicarbonate (3g, 35.71mmol), dichloromethane (60mL) are successively added to the reaction bottle in a nitrogen environment. Cool the reaction mixture to 5C° and slowly add compound 2 (2.58g, 18.58mmol). After the drip is finished, stir the reaction mixture at 5-10°C for 5h. TLC (Thin Layer chromatography) monitoring (Petroleum ether (PE)/ethyl acetate (EA) =4/1) After the reaction is over, the undissolved solids are removed by filtration and the filtrate is collected. The organic phase was washed twice with 1N HCl solution (50mL), then washed with saturated salt water (50mL), and the organic phase was dried with anhydrous magnesium sulfate. Filtration, filtrate concentration to obtain 5.85g colorless oil liquid, without purification, directly for the next reaction.

Step 2: In nitrogen environment, compound 4 (2.95g, 8.72mmol), sodium iodide (3g, 20.11mmol), potassium carbonate (2.78g, 20.11mmol) and N, n-dimethylformamide (40mL) were added to the reaction bottle in sequence. When the mixture is well stirred, compound 3 (2.5g, 6.70mmol) is added at room temperature, and then stirred at room temperature for 3h. After LCMS monitoring the reaction (see Table 1 for monitoring conditions) is finished, the reaction liquid is poured into 400mL of water. The precipitated oil-like insoluble matter was collected and dissolved in 100mL ethyl acetate. The solution was washed with 100mL water and 100mL saturated salt water successively, the organic phase was dried with anhydrous sodium sulfate, filtered and concentrated, and the residue was separated by normal phase preparation chromatography (PE/EA=1/1) to obtain light yellow solid compound 5 (630mg, yield: 14%).

Step 3: Compound 5 (300mg, 0.44mmol), Pd(OH)/C(150mg)₂ and ethanol (5mL) were added to the reaction bottle in sequence. After the atmosphere in the reaction bottle was replaced by hydrogen balloon for 5 times, it was stirred at room temperature for 3h. After the reaction was monitored by LCMS, filtrate was filtered and collected, and filter cake was washed twice with ethanol (2mLx2). The filtrate was combined and directly separated by RP-HPLC (ammonium bicarbonate aqueous solution/acetonitrile system). The separation method is shown in Table 2 below. The prepared solution was lyophilized directly to obtain white solid compound DM-C1 (120 mg, yield: 50%). ¹H NMR (400 MHz, DMSO-*d*₆) δ 10.87 (s, 1H), 7.50 (d, *J =* 7.6 Hz, 1H), 7.36 -- 7.32 (m, 1H), 7.16 -- 6.88 (m, 12H), 6.61 6.51 (m, 1 h), 3.73 3.53 (m, 3 h), 3.27 3.16 (m, 2 h), 3.08 (s, 4 h), 3.02 2.81 (m, 2 h), 2.73 2.68 (m, 3 h), 1.67 1.64 (m, 2 H), 1.38 1.02 (m, 4 H). The LCMS: [m + H]⁺ = 541.27 m/z.

LCMS (detection method is shown in Table 3) and NMR show that the obtained product is a pair of diastereoisomers (molar ratio about 1/1), and the structural formula is as follows:

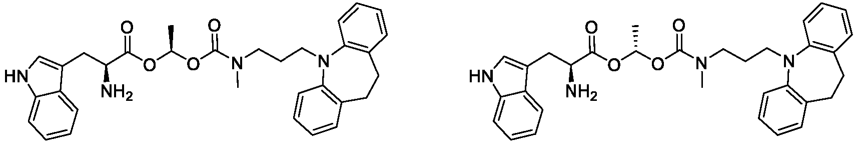

**Table 1 LCMS monitoring methods for monitoring compounds in synthesis**

| Chromatographic column | InfinityLab Poroshell 120 EC-C18 50*2.1mm 2.7µm | System | Aqueous solution containing 0.05% trifluoroacetic acid (TFA) - Acetonitrile (ACN) containing 0.05%TFA |
|---|---|---|---|
| Mobile Phase A | Aqueous solution containing 0.05% TFA | Mobile Phase B | ACN with 0.05% TFA |
| Gradient time (min) | 5% mobile phase B held for 0.1 minutes; Mobile phase B increased from 5% to 95% in 0.8 minutes; 95% of mobile phase B remained for 0.4 minutes; Then at 1.31 minutes mobile phase B was reduced to 5% and held for 0.19 minutes | Flow rate (ml/min) | 1.0 mL/min |
| Detection wavelength | 214nm, 254nm | Column temperature | 40°C |
| MS Ionization (mass spectrometry ionization source) | ESI | MS scan (mass spectrum scan range) | 100-1000 |

**Table 2 RP-HPLC preparative separation method DM-C1**

| Chromatographic Column | YMC-Triart Prep C18 250*50mm*7um | System | Water (10mM ammonium bicarbonate)- acetonitrile system |
|---|---|---|---|
| Mobile Phase A | Water with 10mM ammonium bicarbonate | Mobile phase B | acetonitrile |
| Starting B value | 48% | End B value | 96% |
| Gradient time (min) | 0→30 min: The volume percentage of mobile phase B is from 48% to 96% | Flow rate (ml/min) | 80ml/min |
| Detection wavelength | 220 nm and 254 nm | Column temperature | Room temperature |

**Table 3 LCMS test method for final compounds DM-C1**

| Sample ID | E0033-021-P1 | | |
|---|---|---|---|
| Column | ACQUITY UPLC BEH C18 Ysc-triart Prep C18 50*2.1mm*1.7um | System | Water (0.05% ammonia) -acetonitrile system |
| Mobile Phase A | Water containing 0.05% industrial | Mobile Phase B | acetonitrile |
| | ammonia | | |
| Starting B value | 5% | End B value | 95% |
| Gradient time (min) | 0→7 min: the volume percentage of mobile phase B varies from 5% to 95% | Flow rate (ml/min) | 1ml/min |
| Detection wavelength | 214nm and 254nm | Column temperature | 40 °C |
| MS Ionization | ESI | MS scan | 100-1000 |

### Example 2

This embodiment is used to prepare the compound DM-C2, whose name is 1- ((3- (10, 11-dihydro-5h-dibenzo [b, f] azapyridine-5-yl) propyl) (methyl) carbamyl-oxy) ethyl benzoate, the compound DM-C2 described in this embodiment is synthesized by the following route, which specifically includes the following steps:

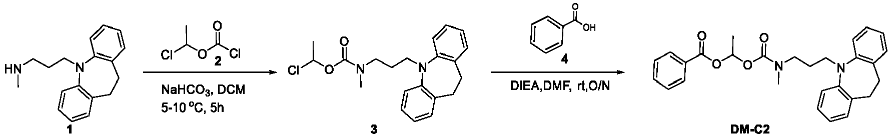

Step 1: In nitrogen environment, compound 1 (10 g, 37.53 mmol), sodium bicarbonate (6.67 g, 79.28mmol), dichloromethane (100 mL) were added to the reaction bottle successively, then the reaction system was cooled to 5C°, and compound 2 (5.90 g, 41.29 mmol) was added by drops. The reaction system was stirred at 5-10°C for 5h. After the reaction, the undissolved sodium bicarbonate solid was removed by filtration, the filtrate was collected, the organic phase was washed twice with 1N HCl solution, and then washed with saturated salt water, the organic phase was dried with anhydrous magnesium sulfate, and the organic phase was concentrated to obtain a colorless oily liquid (13g, yield: 92.8%), without purification, and directly used for the next reaction.

Step 2: Add compound 4 (500 mg, 4.02 mmol),N, n-diisopropyl ethylamine (1.73 g, 13.41 mmol),N, n-dimethylformamide (15 mL) to the reaction bottle in nitrogen environment, stir well, and add compound 3 (1 g, 2.68 mmol) at room temperature. And then stir at room temperature for 16h. After the reaction, DM-C2 was prepared and separated by using reversed-phase high performance liquid (see Table 4). The separated solution was lyophilized to obtain yellow oily liquid (150 mg, yield: 12.2%). The products were tested by LCMS (see Table 5 for the method of picking up) and nuclear magnetic test. ¹H NMR (400 MHz, *Chloroform-d*) δ 8.03 (t, *J* = 6.4 Hz, 2H), 7.59 -- 7.55 (m, 1H), 7.45 -- 7.41 (m, 2H), 7.12 -- 7.02 (m, 7 h), 6.91 (t, *J =* 7.2 Hz, 2 h), 3.76 3.71 (m, 2 h), 3.40 3.23 (m, 2 h), 3.15 (s, 4 h), 2.80 2.77 (m, 3 h), 1.84 1.77 (m, 2 H), 1.60 1.48 (m, 3 H). The LCMS: [m + H]⁺ = 459.2 m/z.

**Table 4 Preparative separation method for DM-C2 by reversed-phase high performance liquid phase**

| Chromatographic Column | Welch Xtimate C18 250*50mm*10um | System | Water (10mM ammonium bicarbonate)- acetonitrile |
|---|---|---|---|
| Mobile Phase A | Water with 10mM ammonium bicarbonate | Mobile phase B | acetonitrile |
| Starting B value | 66% | End B value | 96% |
| Gradient time | 0→22 min: The volume percentage of | Flow rate | 80ml/min |
| (min) | mobile phase B is from 66%-96% | (ml/min) | |
| Detection wavelength | 220 nm and 254 nm | Column temperature | Room temperature |

**Table 5 LCMS test method for final compounds DM-C2**

| Chromatographic column | ACQUITY UPLC BEH C18 Ysc-triart Prep C18 50*2.1mm*1.7um | System | Water (0.05% ammonia) -acetonitrile system |
|---|---|---|---|
| Mobile Phase A | Water with 0.05% ammonia | Mobile Phase B | acetonitrile |
| Gradient time (min) | 5% of the mobile phase B is held for 0.1 minutes; Mobile phase B increased from 5% to 95% in 5.9 minutes; 95% of mobile phase B remained for 0.8 minutes; Then at 6.81 minutes mobile phase B was reduced to 5% and held for 0.19 minutes | Flow rate (ml/min) | 1ml/min |
| Detection wavelength | 214nm, 254nm | Column temperature | 40°C |
| MS Ionization | ESI | MS scan | 100-1000 |

### Example 3

This embodiment is used to prepare the compound DM-C3, which is called (2R) -2-amino-5 - (1-(((3- (10, 11-dihydro-5h-dibenzo [b, f] aza-5-yl) propyl) (methyl) carbamoyl) oxy) ethoxy) -5-oxglutaric acid, The synthesis route of the compound DM-C3 described in the present embodiment is shown as follows, specifically consisting of the following steps:

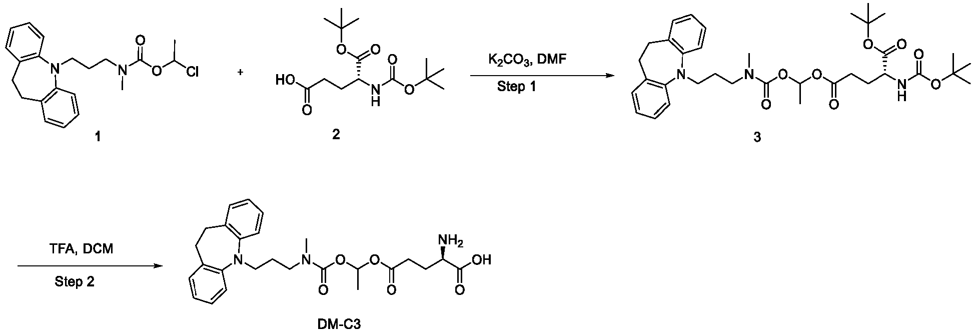

Step 1: In nitrogen, add compound 1 (500 mg, 1.34 mmol), compound 2 (407 mg, 1.34 mmol), potassium carbonate (371 mg, 2.68 mmol), N, N-dimethylformamide (20 mL) to the reaction bottle, stir well, stir at room temperature for 3h. After the reaction, the reaction liquid was poured into 80 mL water, extracted with ethyl acetate (40 mL*3), the organic phase was washed with saturated salt water and water, and concentrated and dried to obtain crude product. The crude product was separated forward (PE:EA=3:1, Rf=0.4) to obtain colorless oil-like compound 3 (710 mg, yield: 12.2%).

Step 2: In nitrogen environment, compound 3 (610 mg, 0.95 mmol) and dichloromethane (16 mL) were added into the reaction bottle successively. After stirring well, trifluoroacetic acid (8 mL) was added at room temperature, and the reaction was stirred at room temperature for 3h. After the reaction is over, concentrate the dry to the crude product directly. The crude product was prepared and separated by reversed-phase high performance liquid phase (trifluoroacetic acid aqueous solution/acetonitrile system). The separation method was shown in Table 6 below. After separation, acetonitrile was concentrated and lyophilized in the separated solution to obtain DM-C3 as a white solid (370 mg, yield: 80.3%). ¹H NMR (400 MHz, Methanol-*d*₄) δ7.12 (dq, *J =* 8.0, 4.5Hz, 6H), 6.93 (m, 2H), 6.71 (m, 1H), 3.96 (dt, *J =* 9.2, 6.7Hz, 1 h), 3.82 3.71 (m, 2 h), 3.39 (dt, *J* = 14.4, 7.2 Hz, 1 h), 3.32 3.24 (m, 1 h), 3.18 (d, *J* = 6.8 Hz, 4 h), 2.85 2.75 (m, 3 h), 2.56 (q, *J* = 7.2 Hz, 2 h), 2.29 2.07 (m, 2 h), 1.81 (2 h p, *J* = 6.8 Hz), 1.47 (d, *J* = 5.4 Hz, 1.2 H), 1.29 (d, *J* = 5.4 Hz, 1.8 H). The LCMS: [m + H]⁺ = 484.3 m/z

LCMS (detection method is shown in Table 7) and NMR show that the resulting product is a pair of diastereoisomers, and the NMR shows that the molar ratio of the two is about 2:3. The structural formula is as follows:

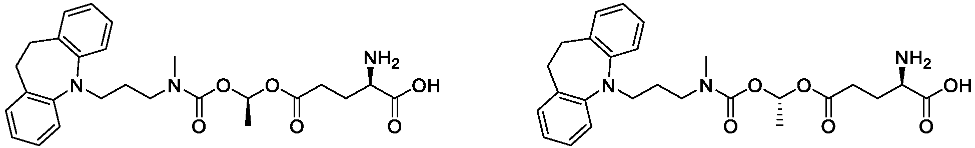

**Table 6 DM-C3 preparative separation method for RP-HPLC**

| Chromatographic column | Welch Xtimate C18 250*50mm*10um | System | Water (0.01% trifluoroacetic acid)-acetonitrile |
|---|---|---|---|
| Mobile Phase A | Water with 0.01% trifluoroacetic acid | Mobile Phase B | acetonitrile |
| Starting B value | 39% | End B value | 69% |
| Gradient time (min) | 0→20 min: The volume percentage of mobile phase B increases from 39% to 69% | Flow rate (ml/min) | 80ml/min |
| Detection wavelength | 220 nm and 254 nm | Column temperature | Room temperature |

**Table 7 LCMS test method for final compounds DM-C3**

| Chromatographic column | ZORBAX Eclipse Plus C18 50*2.1mm 1.8µm | System | Aqueous solution with 0.05%TFA - acetonitrile with 0.05%TFA |
|---|---|---|---|
| Mobile Phase A | Aqueous solution containing 0.05%TFA | Mobile Phase B | Acetonitrile containing 0.05%TFA |
| Gradient time (min) | 5% mobile phase B held for 0.1 minutes; Mobile phase B increased from 5% to 95% in 5.9 minutes; 95% of mobile phase B remained for 0.8 minutes; Then at 6.81 minutes mobile phase B was reduced to 5% and held for 0.19 minutes | Flow rate (ml/min) | 1.0 mL/min |
| Detection | 214nm, 254nm | Column temperature | 40°C |
| wavelength | | | |
| MS Ionization | ESI | MS scan | 100-1000 |

### Example 4

This embodiment is used to prepare the compound DM-D2, whose name is phenyl (3- (10, 11-dihydro-5h-dibenzo [b, f] aza-5-yl) propyl) (methyl) carbamate. The synthesis route of the compound DM-D2 described in this embodiment is shown as follows, specifically consisting of the following steps:

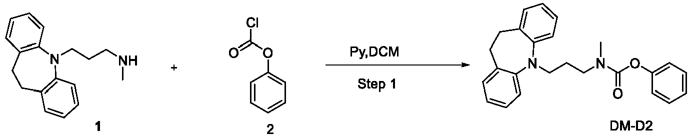

Under nitrogen protection, compound 1 (1 g, 3.75 mmol), dichloromethane (10 mL), pyridine (890.80 mg, 11.26 mmol) are successively added to the reaction bottle, after stirring evenly, Compound 2 (881.62mg, 5.63mmol) was added into the ice bath, and the reaction was stirred for 2h under the ice bath. After the reaction is over, concentrate the dry to the coarse product directly. The crude product was prepared and separated by using normal high performance liquid phase (PE petroleum ether /EA ethyl acetate). The separation method was shown in Table 8 below. The separated solution was spin dried, and 10ml lyophilized acetonitrile and 10ml lyophilized water were added to freeze dry to obtain DM-D2 as colorless oil (870 mg, yield: 59.96%). ¹H NMR (400 MHz, DMSO-*d*₆) δ 7.39-7.25 (m, 2H), 7.22-7.08 (m, 6H), 7.03 (t, *J* = 8.8Hz, 2H), 6.95-6.87 (m, 2H), 6.79 (d, *J =* 8.0 Hz, 1 H), 3.75 (q, *J =* 6.0 Hz, 2 H), 3.44 (t, *J =* 7.0 Hz, 1 H), 3.32 (s, 1 H), 3.10 (s, 1.7 H), 3.07 (s, 2.3 H), 2.88 (s, 1.2 H), 2.86 (s, 1.7 H), 1.77 (m, 2 H). The LCMS: [m + H]⁺ = 387.2 m/z.

**Table 8 Preparative separation method of DM-D2 in normal phase high performance liquid phase**

| Chromatographic Column | 12g silica gel column | System | PE-EA system |
|---|---|---|---|
| Mobile Phase A | PE | Mobile Phase B | EA |
| Starting B value | 0 | End B value | 20% |
| Gradient time (min) | 0→30 min: The volume percentage of mobile phase B is increased from 0 to 20% | Flow rate (ml/min) | 20ml/min |
| Detection wavelength | 254 nm and 280 nm | Column temperature | Room temperature |

**Table 9 LCMS test method for final compounds DM-D2**

| Chromatogra phic column | ZORBAX Eclipse Plus C18 50*2.1mm 1.8µm | system | Aqueous solution with 0.05%TFA - acetonitrile with 0.05%TFA |
|---|---|---|---|
| Mobile Phase A | Aqueous solution containing 0.05%TFA | Mobile Phase B | Acetonitrile containing 0.05%TFA |
| Gradient time | 5% mobile phase B held for | Flow rate (ml/min) | 1.0 mL/min |
| (min) | 0.1 minutes; Mobile phase B increased from 5% to 95% in 5.9 minutes; 95% of mobile phase B remained for 0.8 minutes; Then at 6.81 minutes mobile phase B was reduced to 5% and held for 0.19 minutes | | |
| Detection wavelength | 214nm, 254nm | Column temperature | 40°C |
| MS Ionization | ESI | MS scan | 100-1000 |

### Example 5

This embodiment is used to prepare the compound DM-D3, whose name is (5-methyl-2-oxo-1, 3-dioxole-4-yl) methyl (3- (10, 11-dihydro-5h-dibenzo [b, f] azapyridine-5-yl) propyl) (methyl) carbamate, with the structural formula as follows:

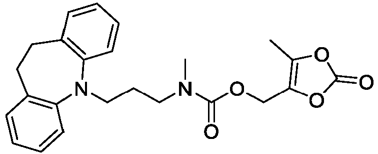

The synthesis route of the compound DM-D3 described in the present embodiment is shown as follows, specifically consisting of the following steps:

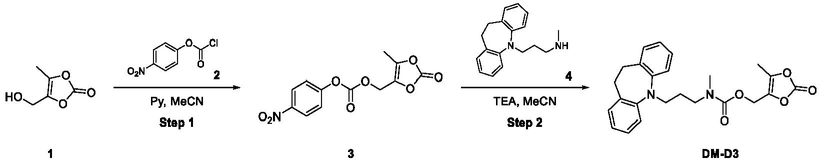

Step 1: Compound 1 (200mg, 1.54mmol), acetonitrile (4mL), pyridine (365mg, 4.61mmol) were added sequentially to the reaction bottle in nitrogen environment. After stirring well, compound 2 (341mg, 1.69mmol) was added at 0°C and slowly returned to room temperature for stirring for 5h. After the reaction, the reaction liquid was directly used in the next step to obtain compound 3 (450mg, yield: 99%, crude).

Step 2: In nitrogen environment, compound 4 (325mg, 1.22mmol) and acetonitrile (3mL) were added into the reaction bottle successively. After stirring well, compound 3 (450mg, 1.52mmol) was added at 0°C and slowly returned to room temperature for stirring for 12h. After the reaction is over, concentrate the dry to the coarse product directly. The crude product was prepared and separated using reversed-phase high performance liquid phase (formic acid aqueous solution/acetonitrile system). The separation method is shown in Table 10 below. After separation, acetonitrile was concentrated in the separated solution and lyophilized directly to obtain DM-D3 yellow oily liquid (200mg, yield: 31%). 1H NMR (400 MHz, Chloroform-d) δ 7.18-7.01 (m, 6H), 6.92 (td, J = 7.4, 1.3Hz, 2H), 4.79 (s, 0.9H), 4.58 (s, 1.2H), 3.74 (t, J = 6.4 Hz, 2 H), 3.29 (dt, J = 10.4, 7.2 Hz, 2 H), 3.17 (d, J = 5.6 Hz, 4 H), 2.79 (s, 1.7 H), 2.74 (s, 1.3 H), 2.14 (d, J = 3.4 Hz, 3 h), 1.79 (dp, J = 13.2, 7.0 Hz, 2 h). The LCMS: [m + 1) + m/z = 423.1.

**Table 10 Preparative separation method of reversed-phase high performance liquid phase DM-D3**

| Chromatographic Column | Phenomenex Luna C18 250*50mm*10um | System | Water (0.225% formic acid)- acetonitrile |
|---|---|---|---|
| Mobile Phase A | Water with 0.225% formic acid | Mobile Phase B | acetonitrile |
| Starting B value | 48% | End B value | 78% |
| Gradient time (min) | 0→20 min: The volume percentage of mobile phase B increases from 48% to 78% | Flow rate (ml/min) | 80ml/min |
| Detection wavelength | 220 nm and 254 nm | Column temperature | Room temperature |

**Table 11 LCMS test method for final compounds DM-D3**

| Chromatogra phic column | ZORBAX Eclipse Plus C18 50*2.1mm 1.8µm | System | Aqueous solution with 0.05%TFA - acetonitrile with 0.05%TFA |
|---|---|---|---|
| Mobile Phase A | Aqueous solution containing 0.05%TFA | Mobile Phase B | Acetonitrile containing 0.05%TFA |
| Gradient time (min) | 5% mobile phase B held for 0.1 minutes; Mobile phase B increased from 5% to 95% in 5.9 minutes; 95% of mobile phase B remained for 0.8 minutes; Then at 6.81 minutes mobile phase B was reduced to 5% and held for 0.19 minutes | Flow rate (ml/min) | 1.0 mL/min |
| Detection wavelength | 214nm, 254nm | Column temperature | 40°C |
| MS Ionization | ESI | MS scan | 100-1000 |

### Example 6

This embodiment is used to prepare the compound DM-D4, whose name is (Phosphonoxy) methyl (3- (10, 11-dihydro-5h-dibenzo [b, f] aza-5-yl) propyl) (methyl) carbamate. The compound DM-D4 described in this embodiment is synthesized by the following route, which consists of the following steps:

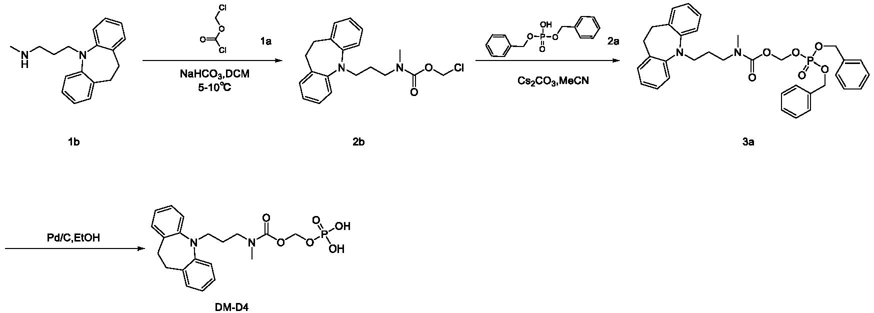

Step 1: In nitrogen environment, compound 1b (500mg, 1.88mmol), sodium bicarbonate (371mg, 2.68mmol) and dichloromethane (5mL) were added to the reaction bottle successively. The reaction system was cooled to 5°C, and compound 1a (266mg, 2.05mmol) was added by drops. After the drip, The reaction system was stirred at 5-10°C for 5h. After the reaction, the undissolved sodium bicarbonate solid was removed by filtration, the filtrate was collected, the organic phase was dried with anhydrous magnesium sulfate, and the organic phase was concentrated to obtain a colorless oil-like liquid (660mg, yield: 97.1%), without purification, and directly used for the next reaction.

Step 2: Compound 2b (600mg, 1.67mmol), compound 2a (558mg, 2.01mmol), cesium carbonate (1.63g, 5.02mmol) and acetonitrile (10mL) were added to the reaction bottle at room temperature, mixed evenly and heated to 50°C for 16h. After the reaction, the coarse product was filtered and concentrated to obtain. The crude product was separated forward (PE:EA=2:1, Rf=0.5) to obtain a colorless oil-like compound 3a (540mg, yield: 53.8%).

Step 3: Compound 3a (540mg, 0.89mmol), ethanol (20mL) and Pd/C (105mg, 0.99mmol) were added to the reaction bottle in order to stir well in the hydrogen environment, and the reaction was carried out at room temperature for 5h. After the reaction, directly concentrate and dry to get the crude product. Direct preparation and separation (formic acid aqueous solution/acetonitrile system) using reversed-phase high performance liquid phase, the separation method is shown in Table 12 below. The separated solution concentrates acetonitrile and lyophilizes it directly to obtain compound DM-D4 as a white solid (220mg, yield: 58.2%). 1HNMR (400 MHz, Chloroform-d) δ 7.37 (s, 3H), 7.07 (h, J = 8.0Hz, 6H), 6.90 (q, J = 7.2Hz, 2H), 5.50 (d, J = 12.8 Hz, 1 h), 5.34 (d, J = 12.8 Hz, 1 h), 3.69 (dt, J = 12.8, 6.5 Hz, 2 h), 3.24 (q, J = 6.4 Hz, 2 h), 3.14 (s, 4 h), 2.71 (d, J = 6.4 Hz, 3 H), 1.75 (p, J = 6.8 Hz, 2 H). The LCMS: [m + H] + m/z = 421.1.

**Table 12 Preparative separation method of reversed-phase high performance liquid phase DM-D4**

| Chromatographic Column | Phenomenex Luna C18 250*50mm*10um | System | Water (0.225% formic acid)- acetonitrile |
|---|---|---|---|
| Mobile phase A | Water with 0.225% formic acid | Mobile Phase B | acetonitrile |
| Starting B value | 30% | End B value | 60% |
| Gradient time (min) | 0→20 min: The volume percentage of mobile phase B increases from 30% to 60% | Flow rate (ml/min) | 80ml/min |
| Detection wavelength | 220 nm and 254 nm | Column temperature | Room temperature |

**Table 13 LCMS test method for final compounds DM-D4**

| Chromatogra phic column | ZORBAX Eclipse Plus C18 50*2.1mm 1.8µm | System | Aqueous solution with 0.05%TFA - acetonitrile with 0.05%TFA |
|---|---|---|---|
| Mobile Phase A | Aqueous solution containing 0.05%TFA | Mobile Phase B | Acetonitrile containing 0.05%TFA |
| Gradient time (min) | 5% mobile phase B held for 0.1 minutes; Mobile phase B increased from 5% to 95% in 5.9 minutes; 95% of mobile phase B remained for 0.8 minutes; Then at 6.81 minutes mobile phase B was reduced to 5% and held for 0.19 minutes | Flow rate (ml/min) | 1.0 mL/min |
| Detection wavelength | 214nm, 254nm | Column temperature | 40°C |
| MS Ionization | ESI | MS scan | 100-1000 |

### Contrast Experiment 1

The pair ratio is used to prepare the compound DM-A1, whose name is (S) -4-amino-5 - ((3- (10, 11-dihydro-5h-dibenzo [b, f] aza-5-yl) propyl) (methyl) amino) -5-oxopentenoic acid, the synthesis route of the compound DM-A1 described in the pair ratio is shown as follows, specifically including the following steps:

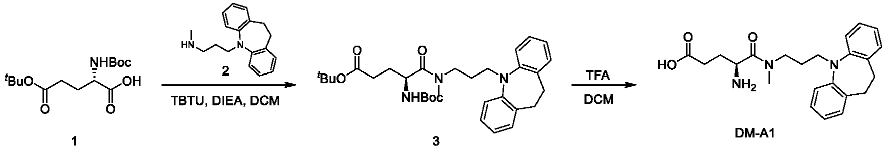

Step 1: In a nitrogen environment, add dichloromethane (15 ml) to the reaction bottle, followed by compound 1 (500 mg,1.65 mmol), 1-[bis (dimethylamino) methylene] -1h-benzotriazolium 3-tetrafluoroborate (1.06 g,3.30 mmol) and N, n-diisopropylethylamine (639 mg,4.94 mmol) were substituted with nitrogen three times. The reaction liquid is stirred at room temperature for 10 minutes. Compound 2 (483 mg,1.81 mmol) is added to the reaction solution and stirred at this temperature for 3 hours. When the reaction is complete, add 50 ml of water and extract twice more with methylene chloride (30 ml each time). The organic phase is washed in saturated salt water, dried and concentrated to obtain the crude product. The crude product was purified by silica gel column chromatography to obtain compound 3 (700 mg, yield: 77%) colorless transparent oil-like product.

Step 2: In nitrogen, add methylene chloride (10 ml) and trifluoroacetic acid (10 ml) to the reaction bottle, and add compound 3 (700 mg, 1.27 mmol). The reaction is stirred at room temperature for 3 hours. After the reaction is complete, concentrate the reaction liquid directly to get the crude product. The crude product was isolated by reversed-phase high performance liquid chromatography (water (0.225% formic acid)/ acetonitrile system to obtain the compound DM-A1 (260 mg, yield: 52%) white solid product. ¹H NMR (400 MHz, DMSO - *d*₆) delta 7.19 7.03 (m, 6 H), 6.96 6.84 (m, 2 H), 3.98 3.59 (m, 3 H), 3.33 (m, 2 H), 2.89 (s, 2 H), 2.69 (s, 1 H), 2.37 2.14 (m, 2 H), 1.69 (m, 3 H), 1.51 (m, 1 H). The LCMS: [m + H]⁺ = 396.2 m/z.

### Experiment 1 Single Dose Pharmacokinetic Study of DM-A1 and Desipramine in SD Rats

Pharmacokinetic experiment: Eighteen SD rats were randomly divided into 3 groups with 6 rats in each group, 180~200g each. The tested drugs were DM-A1 and desipramine hydrochloride, respectively, and the tested drug solution was prepared with carboxymethyl cellulose sodium solution (prepared by mixing 5ml dimethyl sulfoxide and 95ml carboxymethyl cellulose sodium aqueous solution with concentration of 0.5wt%) as the solvent. The dosage and mode of administration were shown in the following table. Fast for at least 12h but not more than 18h before intragastric administration, and give food for 4h after administration; Cannot refrain from drinking water before and after administration. Do not fast before and after the administration of the caudal vein, and cannot refrain from drinking water. The frequency of administration was single administration, and the volume of administration was 5mL/kg.

**Table 14 Preparation of tested drugs**

| **Groups** | **Number of Animals** | **Method of administration** | **subject** | **Dosage (mg/kg)** | **Blood collection time point** |
|---|---|---|---|---|---|
| 1 | 6 | Intragastric adminstration | DM-A1 | 20 | Before (0h) and after administration, 5min, 10min, 20min, 30min, 1h, 2h, 4h, 6h, 12h, 24h |
| 2 | 6 | Intragastric administration | Desipramine hydrochloride | 20 | |
| 3 | 6 | Tail vein injection | Desipramine hydrochloride | 4 | |

Blood samples were collected before (0h) and 5min, 10min, 20min, 30min, 1h, 2h, 4h, 6h, 12h, 24h after administration. Plasma was obtained from the samples and quantified for DM-A1 and desipramine by LC-MS/MS method. The chromatographic conditions were as follows: ACE 5 C18 column, 50×2.1mm; Mobile phase A: 0.1%FA aqueous solution, B: ACN containing 0.1%FA, gradient elution, elution procedure is as follows: 0→0.3min→0.7min→1,5min →1.6min→2.1min, the volume percentage of mobile phase A is 70%-70%-5%-5%-70%-70%, the volume percentage of mobile phase B is
30%-30%-95%-95%-30%-30%, and the column temperature is 45°C. The flow rate was 0.4mL/min. The MS conditions are as follows: Ionization mode of DM-A1 is Postive/MRM, Q1/Q3 is 396.3/378.2, DP is 70V, CE is 15V. The ionization mode of desipramine hydrochloride is Postive/MRM, Q1/Q3 is 266.9/236.2, DP is 70V, CE is 21V.

The following pharmacokinetic parameters of DM-A1 and desipramine were calculated: area under blood concentration-time curve (AUC₀₋ₜ), peak concentration (Cₘₐₓ), peak time (Tₘₐₓ), half-life (t_{1/2}), clearance rate (CL), bioavailability (F).

The experimental results are shown in the following table:

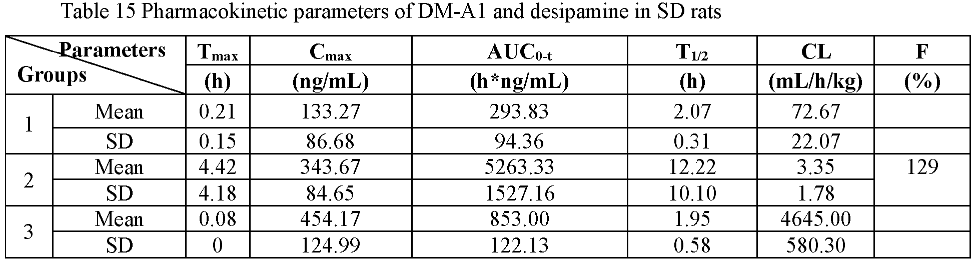

Results: Desipramine was not detected in plasma of SD rats given DM-A1 by gavage, so the relevant parameters of desipramine were not evaluated and calculated in group DM-A1 (group1, group1). The table shows the relevant parameters of DM-A1. Desipramine was detected in both desipramine intragastric and intravenous administration groups (groups 2 and 3).

### Experiment 2 Single Dose Pharmacokinetics Study of DM-C2, DM-C3 and Desiparmine in SD Rats

Pharmacokinetic experiment: Fourteen SD rats were randomly divided into 3 groups with 6 rats in one group and 4 rats in the other two groups with 180-200g in each group. The tested drugs were DM-C2, DM-C3 and desipramine hydrochloride, respectively. Sodium carboxymethyl cellulose solution (prepared by mixing 5ml dimethyl sulfoxide and 95ml sodium carboxymethyl cellulose solution with a concentration of 0.5wt%) was used as the solvent to prepare the tested drug solution. The dosage and method of administration were shown in the following table. Fasting for at least 12h but not more than 18h before intragastric administration, and feeding for 4h after administration; Cannot refrain from drinking water before and after administration. The frequency of administration was single dose, and the volume of administration was 10mL/kg.

**Table 16 Preparation of tested drugs**

| **group** | **Number of Animals** | **Method of administration** | **subject** | **Dosage (mg/kg)** | **Blood collection time point** |
|---|---|---|---|---|---|
| 1 | 6 | Gavage | Desipramine hydrochloride | 10 | Before (0h) and after administration, 5min, 10min, 20min, 30min, 1h, 2h, 4h, 6h, 12h, 24h |
| 2 | 4 | Administration by gavage | DM-C2 | 15 | |
| 3 | 4 | Intragastric administration | DM-C3 | 21 | |

The dose of desipramine hydrochloride was used as the benchmark for dose conversion in this experiment, and the other two subjects were converted with the same molar amount of desipramine. Factors such as salt type and purity were taken into account in the calculation process.

Blood samples were collected before (0h) and 5min, 10min, 20min, 30min, 1h, 2h, 4h, 6h, 12h, 24h after administration. Plasma was obtained from the samples and quantified for DM-C2, DM-C3 and desipramine by LC-MS/MS method. The chromatographic conditions were as follows: XB-C8 (2.1 mm ×50mm, 5 µm), Welch; Mobile phase A: 0.1%FA aqueous solution, B: ACN containing 0.1%FA, gradient elution, elution procedure is as follows: 0.01min→1min→3min→4.5min →4.51min→5.5min, the volume percentage of mobile phase A is 80%-80%-2%-2%-80%-80%, the volume percentage of mobile phase B is 20%-20%-98%-98%-20%-20%, and the column temperature is 45°C. The flow rate was 0.5mL/min. The MS conditions are as follows: The ionization mode of DM-C2 is Positive/MRM, Q1/Q3 is 459.43/234.3, DP is 58V, CE is 30V. The ionization mode of DM-C3 is Positive/MRM, Q1/Q3 is 484.4/311.4, DP is 90V and CE is 22V. The ionization mode of desipramine hydrochloride was Postive/MRM with 267.4/71.9 for Q1/Q3, 80V for DP and 20V for CE.

The following pharmacokinetic parameters of desipramine were calculated: AUC₀₋ₜ, peak concentration (Cₘₐₓ), peak time (Tₘₐₓ), half-life (t_{1/2}), clearance rate (CL).

The experimental results are shown in the following table:

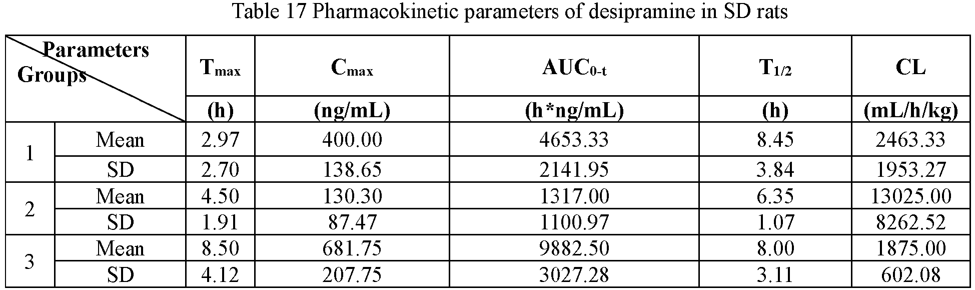

Results: DM-C2 and DM-C3 were not detected in plasma samples of DM-C2 and DM-C3 groups, so their related parameters were not evaluated and calculated. However, desipramine could be detected in DM-C2 and DM-C3 groups. The table shows the parameters of desipramine. In particular, the AUC₀₋ₜ (9882.5h *ng/mL) and C ₘₐₓ(681.75ng /mL) values of DM-C3 group were higher than those of desipramine hydrochloride group ₀₋ₜ(4653.33h *ng/mL) and C ₘₐₓ(400 ng/mL). It is concluded that DM-C3 can increase the systemic exposure of desipramine in SD rats.

### Experiment 3 Single Dose Pharmacokinetics Study of DM-C3 and Desiprmine in SD Rats

Pharmacokinetic experiment: Eighteen SD rats were randomly divided into 3 groups with 6 rats in each group, 180~200g each. The tested drugs were DM-C3 and desipramine hydrochloride, respectively, and the tested drug solution was prepared using sodium carboxymethyl cellulose solution (prepared by mixing 5ml dimethyl sulfoxide and 95ml sodium carboxymethyl cellulose solution with concentration of 0.5wt%) as the solvent. The dosage and method of administration were shown in the following table. Fasting for at least 12h before intragastric administration, and resuming feeding for 4h after administration; Cannot refrain from drinking water before and after administration. The frequency of administration was single administration, and the volume of administration was 10mL/kg, 6 mL/kg, 2 mL/kg according to the order of the group.

**Table 18 Preparation of tested drugs**

| **Groups** | **Number of Animals** | **Method of administration** | **subject** | **Dosage (mg/kg)** | **Blood collection time point** |
|---|---|---|---|---|---|
| 1 | 6 | Gavage | DM-C3 | 32 | Before (0h) and after 10min, 20min, 30min, 1h, 2h, 4h, 6h, 12h, 24h, 48h |
| 2 | 6 | Administer the drug intragastric | Desipramine hydrochloride | 15 | |
| 3 | 6 | Intravenous | Desipramine hydrochloride | 5 | Before administration (0h) and after administration, 5min, 10min, 20min, 1h, 2h, 4h, 6h, 12h, 24h |

The dose conversion of this experiment was based on the dose of desipramine hydrochloride administered by inadministration, and the dose conversion of DM-C3 was carried out with the same molar amount of desipramine. Factors such as salt type and purity were taken into account in the calculation process.

Blood samples were collected by intravenous injection before (0h) and 5min, 10min, 20min, 1h, 2h, 4h, 6h, 12h, 24h after administration; Intragastric administration was performed before (0h) and after 10min, 20min, 30min, 1h, 2h, 4h, 6h, 12h, 24h, 48h. Plasma was obtained from the samples and quantified for DM-C3 and desipramine by LC-MS/MS method. The chromatographic conditions were as follows: YMC Triart C18 column, 5µm 50×2.0mm; Mobile phase A: 0.1%FA (formic acid) aqueous solution, B: ACN containing 0.1%FA (formic acid), gradient elution, elution procedure is as follows: 0→1.0min→1.8min→1.9min →2.6min, the volume percentage of mobile phase B was
30%-95%-95%-30%-stop, the column temperature was 35°C, and the flow rate was 0.8mL/min. MS conditions are as follows: the ionization mode of DM-C3 is Postive/MRM, Q1/Q3 is 484.5/311, DP is 98V, CE is 23V. The ionization mode of desipramine hydrochloride is Postive/MRM, Q1/Q3 is 267.2/236.3, DP is 40V, CE is 22V.

The following pharmacokinetic parameters of desipramine were calculated: area under blood concentration-time curve (AUC₀₋ₜ), peak concentration (Cₘₐₓ), time to peak (Tₘₐₓ), mean residence time (MRTₗₐₛₜ), bioavailability (F%).

The experimental results are shown in the following table:

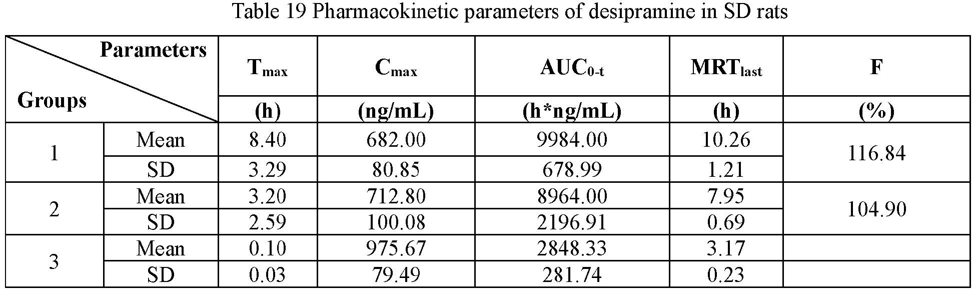

Results: DM-C3 was not detected in plasma samples of DM-C3 group, so its related parameters were not evaluated and calculated. The AUCₗₐₛₜ (9984 h*ng/mL) and Cₘₐₓ (682 ng/mL) in the DM-C3 group were higher than those in the oral desipramine hydrochloride groupₗₐₛₜ (8964 h*ng/mL) and Cₘₐₓ (712.8 ng/mL). The absolute bioavailability of DM-C3 group (116.84%) was higher than that of oral desipramine hydrochloride group (104.9%).

DM-C3 was not detected in the samples of the DM-C3 group, so the relevant parameters of DM-C3 were not evaluated.

Animals No. 2 and No. 8 in the oral DM-C3 and oral desipramine hydrochloride groups showed abnormal values, which were not included in the parameter calculation due to the detection bias caused by individual differences of animals.

In conclusion, after oral administration of DM-C3 in SD rats, the content of desipramine produced by metabolism and entered the systemic circulation was higher than that produced by oral desipramine hydrochloride, indicating that DM-C3 can increase the systemic exposure of desipramine in SD rats and improve its oral bioavailability.

### Example 4 Single Dose Pharmacokinetic Study of DM-D4 and Desipramine in SD Rats

Pharmacokinetic experiment: Twelve SD rats were randomly divided into 2 groups with 6 rats in each group, 180~200g each. The tested drugs were DM-D4 and desipramine hydrochloride, respectively, and the tested drug solution was prepared using sodium carboxymethyl cellulose solution (prepared by mixing 5ml dimethyl sulfoxide and 95ml sodium carboxymethyl cellulose solution with concentration of 0.5wt%) as the solvent. The dosage and method of administration were shown in the following table. Fast for at least 12h but not more than 18h before intragastric administration, and give food for 4h after administration; Cannot refrain from drinking water before and after administration. The frequency of administration was single dose, and the volume of administration was 10mL/kg.

**Table 20 Preparation of tested drugs**

| **group** | **Number of Animals** | **Method of administration** | **subject** | **Dosage (mg/kg)** | **Blood collection time point** |
|---|---|---|---|---|---|
| 1 | 6 | Gavage | Desipramine hydrochloride | 10 | Before (0h) and after 10min, 20min, 30min, 1h, |
| 2 | 6 | Administration by gavage | DM-D4 | 18 | 2h, 4h, 6h, 12h, 24h |

In this experiment, the dose of desipramine hydrochloride was used as the benchmark, and the same molar amount of desipramine was used to convert the dose of DM-D4. Factors such as salt type and purity were taken into account in the calculation process.

Blood samples were collected before (0h) and 10min, 20min, 30min, 1h, 2h, 4h, 6h, 12h, 24h after administration. Plasma was obtained from the samples and quantified for DM-D4 and desipramine by LC-MS/MS method. The chromatographic conditions were as follows: ACE Excel 5 C18, 2.1x50 mm, 5 µm; Mobile phase A: aqueous solution of 0.1%FA and 5mM NH4FA (ammonium formate), B: ACN containing 0.1%FA, gradient elution, elution procedure is as follows: 0.01min→0.5min→1.2min→2.0 min→ 2.1min→3.0min, the volume percentage of mobile phase A is 65%-65%-5%-5%-65%-65%, the volume percentage of mobile phase B is 35%-35%-95%-95%-35%-35%, and the column temperature is 40°C. The flow rate was 0.5mL/min. The MS conditions are as follows: Ionization mode of DM-D4 is Postive/MRM, Q1/Q3 is 421.3/234.2, DP is 80V, CE is 35V. The ionization mode of desipramine hydrochloride is Postive/MRM, Q1/Q3 is 267.2/72.2, DP is 80V, CE is 30V

The following pharmacokinetic parameters of desipramine were calculated: area under blood concentration-time curve (AUC_{0-∞0-t} and AUC), peak concentration (Cₘₐₓ), peak time (Tₘₐₓ), half-life (t_{1/2}), clearance rate (CL).

The experimental results are shown in the following table:

**Table 21 Pharmacokinetic parameters of desipramine in SD rats**

| **Dose Drug** | **Group** | **Dose** | **Sex** | **Animal_ID** | **Tmax (h)** | **Cₘₐₓ (ng/mL)** | **AUC₀₋ₜ (h*ng/mL)** | **T_{1/2} (h)** | ***CL* (mL/h/kg)** |
|---|---|---|---|---|---|---|---|---|---|
| DXPM | 1 | 10 mg/kg | M | 1101 | 4 | 135 | 1270 | NA | NA |
| | | | | 1102 | 1 | 104 | 1300 | 6.1 | 7310 |
| | | | | Mean | 2.5 | 120 | 1280 | 6.1 | 7310 |
| | | | | SD | 2.12 | 21.9 | 23.3 | NA | NA |
| DXPM | 1 | 10 mg/kg | F | 2101 | 1 | 289 | 2660 | 4.5 | 3670 |
| | | | | 2102 | 1 | 164 | 2350 | 6.1 | 3970 |
| | | | | Mean | 1 | 227 | 2500 | 5.3 | 3820 |
| | | | | SD | 0 | 88.4 | 222 | 1.09 | 212 |
| DM-D4 | 2 | 18 mg/kg | M | 1401 | 0.5 | 213 | 1190 | 11 | 7600 |
| | | | | 1402 | 2 | 252 | 2100 | 12 | 4090 |
| | | | | Mean | 1.3 | 233 | 1640 | 12 | 5850 |
| | | | | SD | 1.06 | 27.6 | 638 | 1.01 | 2490 |
| DM-D4 | 2 | 18 mg/kg | F | 2401 | 2 | 190 | 3200 | 7.7 | 5070 |
| | | | | 2402 | 12 | 235 | 4730 | 22 | 2090 |
| | | | | Mean | 7 | 213 | 3960 | 15 | 3580 |
| | | | | SD | 7.07 | 31.8 | 1080 | 9.98 | 2110 |

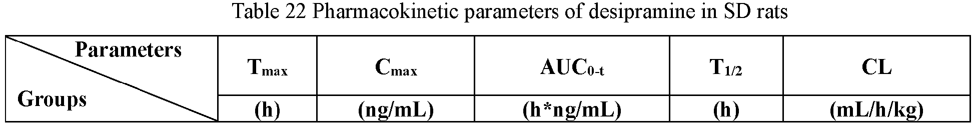

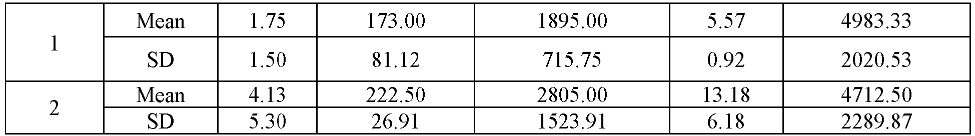

As a result, DM-D4 was not detected in the DM-D4 group, so its related parameters were not evaluated and calculated. AUC0-t (2805 h*ng/mL) and Cmax (222.5 ng/mL) of DM-D4 group were higher than those of desipramine hydrochloride group (1895 h*ng/mL) and Cmax (173 ng/mL). After oral administration of DM-D4, the content of desipramine produced by metabolism and entered the systemic circulation of SD rats was higher than that produced by oral desipramine hydrochloride, indicating that DM-D4 can increase the systemic exposure of desipramine in SD rats and improve its oral bioavailability.

Obviously, the above embodiments are only examples for clear illustration and are not limitations on embodiments. Other variations or variations of different forms may be made on the basis of the above description to persons of ordinary skill in their field. There is no need and cannot be an exhaustive list of all the ways in which they may be carried out. Any apparent variation or alteration resulting therefrom remains within the scope of protection of the invention.

## Claims

1. A compound that increases the bioavailability of desipramine, and its structural formula: 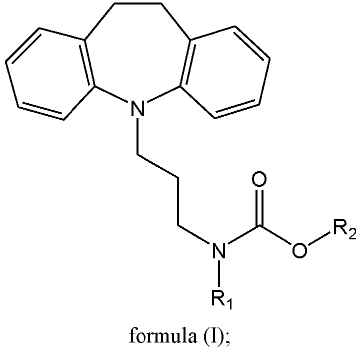
where R₁ is chosen from H, alkyl of C1-C4, alkoxy of C1-C4, or cycloalkyl of C3-C6; R₂ is selected from an unsubstituted or substituted alkyl of C1-C4, an aryl of C5-C20, or 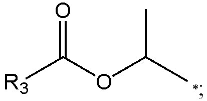
R₃ is selected from the unsubstituted or substituted alkyl of C1-C4 or the aryl of C5-C20;
in the group definition of R₂ and R₃, "substituted" means that 1-3 H of the group is replaced by at least one of the selected groups from hydroxyl, amino, carboxyl, unsubstituted or substituted alkyl of C1-C4, unsubstituted or substituted aryl of C5-C20, unsubstituted or substituted 3 to 20 membered heterocyclic groups, unsubstituted or substituted 3 to 20 membered gelled groups formed by heterocyclic groups and
aromatic rings of C5-C20 and 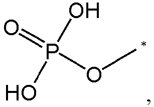
among them, the substituent group of the substituted alkyl group of C1-C4, the aryl group of C5-C20, the heterocyclic group of 3~20 member, the cohesive group formed by heterocyclic group of 3~20 member and the aromatic ring of C5-C20 is alkyl or hydroxyl group of C1-C4; the heteroatom of the heterocyclic group is N, O or S, and the number of heteroatoms is 1-6.

2. According to the compound which improves the bioavailability of desipramine described in claim 1, it is characterized that R₂ is selected from the unsubstituted or substituted alkyl of C1-C4, phenyl or 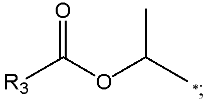 where the term R₃ is as defined in claim 1.

3. The compounds which improve the bioavailability of desipramine described in claim 2 are **characterized by** R₂ being selected from an unsubstituted or substituted alkyl group C1-C4, "substituted" means that the 1-3 H of the alkyl group is replaced by at least one of the hydroxyl groups, 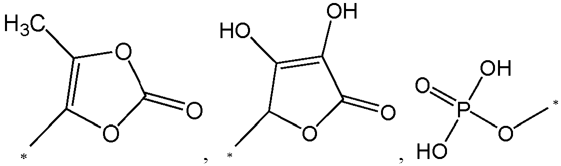 and preferably, R₂ is selected from the methylene group substituted by

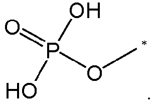

4. Compounds which improve the bioavailability of desipramine according to claim 1 are **characterized in that** the compounds have the structural formula shown in formula II: 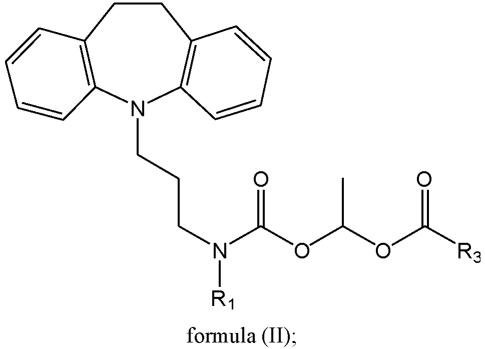 R₁ and R₃ are defined as claim 1.

5. Compounds which improve the bioavailabilty of desipramine described in claim 4 are **characterized in that** R₃ is selected from an unsubstituted or substituted alkyl or phenyl group of C₁-C₄, and substituted means that 1-3 H of the alkyl group is replaced by at least one of the amino group, carboxyl group or 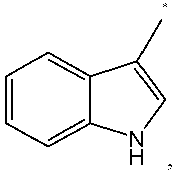 preferably by an amino group or carboxyl group.

6. Compounds which improve the bioavailabilty of desipramine according to claim 1-5 are characterized that R₁ is selected from an alkyl group of H, C1-C4, preferably a methyl group.

7. The compound which improves the bioavailabilty of desipramine according to claim 1 is **characterized by** the selection of the compound from any of the following compounds:

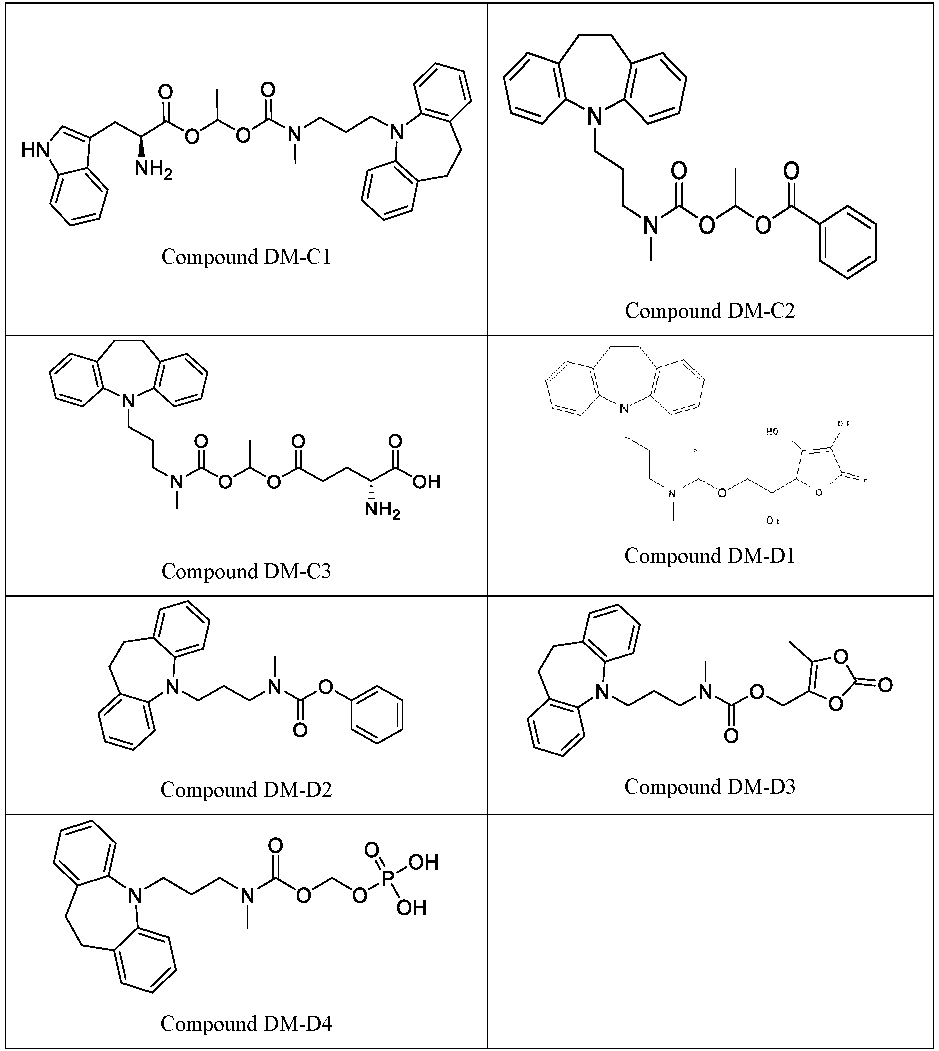

8. A pharmaceutical composition consisting of a compound which improves the bioavailability of desipramine, and pharmaceutically acceptable carriers and/or adjuvants as described in claim 1-7.

9. A method of preventing, treating, or relieving depression, pain, dry macular degeneration, and neuropathy, including the administration of a compound improving the bioavailability of desipramine as described in claim 1-7.

10. The use of the compound improving the bioavailability of desipramine as described in claim 1-7 in the preparation of drugs intended to enhance the bioavailability of desipramine or its pharmaceutically acceptable salts.

11. A compound as defined in any one of claims 1-7 or a pharmaceutical composition according to claim 8 for use in a method of preventing, treating, or relieving depression, pain, dry macular degeneration, or neuropathy.

12. A compound as defined in any one of claims 1-7 or a pharmaceutical composition according to claim 8 for use in a method of enhancing the bioavailability of desipramine or its pharmaceutically acceptable salts.

13. The compound or pharmaceutical composition for use according to claim 11 or claim 12, wherein said method comprises administering desipramine or a pharmaceutically acceptable salt thereof.
